(19) Europäisches Patentamt
European Patent Office
Office européen des brevets

(11) Publication number: **0 081 993**
**B1**

(12) **EUROPEAN PATENT SPECIFICATION**

(45) Date of publication of patent specification: **17.08.88**

(21) Application number: **82306603.0**

(22) Date of filing: **10.12.82**

(51) Int. Cl.[4]: **C 07 C 51/00,** C 07 C 59/64, C 07 C 69/712, C 07 C 67/475, C 07 C 45/00, C 07 C 49/84, C 07 C 43/307, C 07 C 43/295, C 07 C 143/68

(54) **Optically active alpha-substituted aryl ketones, their preparation and their use in preparing alpha-arylalkanoic acids.**

(30) Priority: **11.12.81 US 329672**

(43) Date of publication of application:
**22.06.83 Bulletin 83/25**

(45) Publication of the grant of the patent:
**17.08.88 Bulletin 88/33**

(84) Designated Contracting States:
**AT BE CH DE FR GB IT LI LU NL SE**

(56) References cited:
**EP-A-0 034 871**
**EP-A-0 035 305**
**EP-A-0 048 136**
**EP-A-0 067 698**
**FR-A-2 377 370**
**GB-A-2 026 480**
**US-A-3 637 767**

**CHEMICAL ABSTRACTS, vol. 94, no. 25, 22 June 1981, p. 569, abstract 208547g**
**ULLMANNS ENZYKLOPÄDIE DER TECHNISCHEN CHEMIE, vol. 17, 4th ed., 1979, Weinheim /DE); "Optische aktive Verbindungen", pp. 451-457**
**Interscience Publishers, London-New York-Sydney-Toronto; S.PATAI: "The chemistry of the carbonyl group. Chapter 6: Carbonyl syntheses through organometallics", 1966, pp. 303-304**

(73) Proprietor: **SYNTEX PHARMACEUTICALS INTERNATIONAL LIMITED**
**Global House Church Street**
**Hamilton 5 (BM)**

(72) Inventor: **Schloemer, George C.**
**Blue Mt. Road, P.O. Box 804**
**Lyons, CO 80540 (US)**

(74) Representative: **Armitage, Ian Michael et al**
**MEWBURN ELLIS & CO. 2/3 Cursitor Street**
**London EC4A 1BQ (GB)**

(56) References cited:

**CHEMICAL ABSTRACTS, vol. 92, no. 13, 31 March 1980, p. 634, abstract 110735a, Columbus, Ohio (US)**

**CHEMICAL ABSTRACTS, vol. 89, no. 21, 20 November 1978, p. 584, abstract 179745j, Columbus, Ohio (US)**

**EP 0 081 993 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

**SYNTHESIS, INTERNATIONAL JOURNAL OF METHODS IN SYNTHETIC ORGANIC CHEMISTRY, no. 7, July 1979, Stuttgart (DE); K.OGURA et al.: "A new synthesis of 2-arylpropanoates using methyl methylthiomethyl sulfoxide", pp. 880-882**

## Description

### BACKGROUND OF THE INVENTION

Field of the Invention

The present invention relates to a process for preparing pharmaceutically useful α-arylalkanoic acids. In particular, it relates to a stereoselective process for producing optically active α-arylalkanoic acids which are substantially optically pure.

State of the Art

Numerous α-arylalkanoic acids (i.e. 2-arylalkanoic acids) have been described, developed and found to be useful as pharmaceutical agents exhibiting anti-inflammatory, analgesic and anti-pyretic activity. For example, U.S. Patent No. 3,385,386, describes certain 2-phenylpropionic acids useful for their anti-inflammatory activity. Particularly noteworthy of the compounds described therein is 2-(4-isobutylphenyl)propionic acid, known generically as ibuprofen. U.S. Patent No. 3,600,437 describes 2-(3-phenoxyphenyl)- and 2-(3-phenylthiophenyl)alkanoic acids among other related compounds. Particularly noteworthy therein is the compound 2-(3-phenoxyphenyl)propionic acid, which is known generically as fenoprofen. U.S. Patent No. 3,624,142 describes (fluoro-substituted biphenyl)alkanoic acids, among which is 2-(4′-fluoro-4-biphenylyl)propionic acid. U.S. Patent No. 3,755,427 describes additional fluoro-substituted biphenylpropionic acids, among which is 2-(2-fluoro-4-biphenylyl)propionic acid, known as flurbiprofen. U.S. Patent No. 3,904,682 describes the compound 2-(6-methoxy-2-naphthyl)propionic acid, the d-isomer of which is known generically as naproxen and is a potent anti-inflammatory compound. Related compounds are described in Belgian Patent No. 747,812. U.S. Patent No. 3,912,748 describes 5- and 6-benzoxyazoylalkanoic acids possessing anti-inflammatory, anti-pyrretic and analgesic activity. Notable among those compounds is 2-(4-chlorophenyl-5-benzoxazoyl)propionic acid, known generically as benoxaprofen. Thus, it can be seen that a tremendous variety of useful α-arylalkanoic acids are known.

Other known, useful α-arylalkanoic acids are exemplified by 6-chloro-α-methyl-9H-carbazole-2-acetic acid (carprofen), α-methyl-9H-fluorene-2-acetic acid (cicloprofen), 3-chloro-α-methyl-4-(2-thienylcarbonyl)benzene acetic acid (cliprofen), α-methyl-3-phenyl-7-benzofuranacetic acid (furaprofen), 4-(1,3-dihydro-1-oxo-2H-isoindol-2-yl)benzene acetic acid (indoprofen), 3-benzoyl-α-methylbenzene acetic acid (ketoprofen), 3-chloro-4-(2,5-dihydro-1H-pyrol-1-yl)benzeneacetic acid (pirprofen), α-methyl-4-(2-thienylcarbonyl)benzeneacetic acid (suprofen) and compounds related thereto. Additionally, certain pyrethroid-type insecticides utilize optically active α-arylalkanoic acids, e.g. α-(4-chlorophenyl)isovaleric acid, α-(4-difluoromethoxyphenyl)isovaleric acid and the like, in their formulations.

Numerous processes for the manufacture of such α-arylalkanoic acids have also been described. Such processes have been described in the aforementioned patents, in other patents and in the non-patent literature as well. For example, U.S. Patent No. 4,135,051 describes a process for preparing the ester precursors of many of the useful arylalkanoic acids utilizing trivalent thallium salt as reactants. Such a process suffers from the disadvantage that the thallium salts employed are toxic chemicals which must be removed from the final product. U.S. Patent No. 3,975,431 describes the preparation of α-arylalkanoic acids from glycidonitriles through enol acylates. U.S. Patents Nos. 3,658,863; 3,663,584; 3,658,858; 3,694,476; and 3,959,364 describe various coupling methods for preparing arylalkanoic acids. More recently, U.K. Patent publication No. 2,042,543 published September 24, 1980, (corresponding to application Serial No. 8005752, filed February 20, 1980) describes a process for preparing the ester precursor of arylalkanoic acids from α-haloalkyl aryl ketones using a metal catalyst for catalytically inducing rearrangement in an acidic, alcoholic medium, the catalyst being silver (I) salts of organic and/or inorganic anions. The high costs associated with utilizing metal catalysts, particularly silver, in a large scale process is an inherent disadvantage to such a process. European Patent Application No. 81200210.3, filed February 23, 1981 (Publication No. 0034871, published September 2, 1981) describes a process for preparing esters of α-arylalkanoic acids via rearrangements of α-haloketals in the presence of a Lewis acid (including, for example, copper and zinc salts and the like). Additionally, a recent article in Tetrahedron Letters, Vol. 22, No. 43, pp 4305—4308 (1981) describes a process for producing α-arylalkanoic acids by 1,2-rearrangement of the aryl group via hydrolysis of α-sulfonyloxy acetals.

While the aforesaid processes are useful in many respects, there remains a need for a simple, economical process for producing α-arylalkanoic acids of the types described. Furthermore, in view of the optically active nature of numerous of the α-arylalkanoic acids, it is advantageous to have a stereoselective process for producing the desired optically active isomer of the α-arylkanoic acids which displays all or the major portion of the pharmaceutical activity. For example, the isomer d 2-(6-methoxy-2-naphthyl) propionic acid is more pharmaceutically active than the corresponding l-isomer, and, accordingly, it is desirable to have a stereoselective process for producing the d-isomer directly. Such a process obviates the necessity of subsequently resolving the d- and l-isomers. The elimination of the resolution step results in substantial economic savings, both in material cost and manufacturing labor and plant usage. These savings are particularly significant with regard to those compounds which are approved for pharmaceutical use as a substantially pure, optically active isomer [e.g. d 2-(6-methoxy-2-naphthyl)propionic acid].

Various method are known which employ organometallic reagents in the preparation of carbonyl compounds such as ketones. For example, GB 2026480, Chemical Abstracts 94:208547g and "The

Chemistry of the Carbonyl Group" Chapter 6, by S. Patai, discuss such methods, but are not concerned with the synthesis of optically active ketones, nor with the use of optically active reagents in the synthesis.

EP—A—67698, relevant under Art. 54(3) EPC, discloses a variety of optically active 1-aromatic group substituted 1-alkanones which have an asymmetric carbon atom alpha to the carbonyl group. The aromatic group is introduced by reaction of an aromatic compound with an optically active alkane acyl halide in the presence of a Lewis acid. The optically active 1-aromatic group substituted 1-alkanones are used to prepare optically active α-aryl alkanoic acids through ketalization and rearrangement.

EP—A—64394 and EP-48136, also relevant under Art. 54(3)EPC, describe ketalization of ketones and rearrangement to form carboxylic acids, but do not address the problem of how optically active carboxylic acids might be formed from optically active ketones. EP—A—64394 teaches the resolution of the racemic ketals to afford the optically active enantiomers which can be rearranged to afford the optically active α-aryl-alkanoic acids. Similar processes for preparing esters of α-arylalkanoic acids from α-sulfonyloxyketals or α-haloketals are disclosed in Tetrahedron Letters Vol. 22 pp 4305—4308 (1981) and EP—A—34871 respectively but again, stereoselectivity of the rearrangement is not contemplated and the starting materials are prepared by non-stereoselective methods.

## SUMMARY OF THE INVENTION

The present invention is directed to a process for producing an optically active α-arylalkanoic acid or an ester, ortho ester or amide thereof comprising contacting an organometallic compound, e.g. an aryl magnesium Grignard reagent, with an acyl halide, an acyl amine or an acid anhydride, the acyl halide, acyl amine or acid anhydride being substituted with a leaving group or a group which can be converted into a leaving group. In one aspect, the present invention comprises contacting an aryl organometallic halide with an optically active α-substituted acyl halide, acyl amine or acid anhydride to form the corresponding optically active aryl alkyl ketone, wherein the α-substituent is a leaving group. The ketone group then is ketalized and the substrate formed is rearranged and hydrolyzed to the desired optically active α-arylalkanoic acid. During the rearrangement step of the process, the leaving group disassociates from the substrate and the aryl group migrates to the α-position to afford the rearranged, optically active α-arylalkanoic acid. That aspect of the invention is exemplified by the contact of an aryl magnesium Grignard reagent with an optically active α-sulfonyloxy acyl halide to form the corresponding aryl α-sulfonyloxyalkyl ketone.

The optically active ketones and ketals so produced represent an additional aspect of the present invention. By alternative processes, those ketones can be converted into the desired optically active α-arylalkanoic acids listed previously.

In one embodiment of the invention, the optically active ketones produced are subjected to ketalization under conditions which are amenable to retaining the desired stereochemical configuration at the asymmetric carbon atom of the ketone. For example, ketalization of the ketone with an ortho ester under conditions of acid catalysis affords the desired optically active ketal with retention of the desired configuration at the asymmetric carbon atom. Subsequent solvolytic rearrangement of the ketal yields the desired α-arylalkanoic acid, or the ester, ortho ester or amide thereof. By appropriate choice of the optical configuration of the substituted acyl haide, or the acyl amine or acid anhydride, in view of the single inversion during the rearrangement step, it is possible to produce a desired optically active α-arylalkanoic acid.

The optically active ketone described above can also be subjected to a ketalization process which results in inversion of configuration at the asymmetric carbon atom of the ketone. For example, treatment of the ketone with alkali metal alkoxides or aryloxides typically affords a ketal in which the absolute configuration at the asymmetric carbon atom has inverted. Subsequent solvolytic rearrangement results in an additional inversion at the asymmetric carbon atom to produce the other optically active isomer of the desired α-arylalkanoic acid, or an ester, ortho ester or amide thereof, assuming that the starting ketone in both instances is the same. However, by appropriately choosing the absolute configuration of the starting acyl halide, acyl amine or acid anhydride, it is possible to cause the reaction sequence to yield the desired α-arylalkanoic acid in each instance.

The optically active compounds used and produced by the present invention are preferably a single stereoisomer in the substantial absence of any other stereoisomer thereof.

In one aspect, the present invention is directed to a process for producing an optically active compound of the formula:

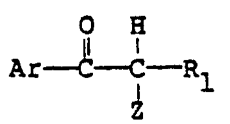

(III)

which comprises converting an organometallic compound of the formula:

ArMX, $(Ar)_2M$ or ArM′

with an optically active acyl halide, an acyl amine or an acid anhydride of the formula

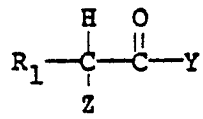 (II)

wherein Ar is aryl, M is cadmium, copper(II), manganese, magnesium or zinc, M' is copper(I) or lithium, $R_1$ is alkyl or cycloalkyl, X is halogen, Y is halogen or a group of the formula:

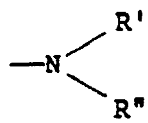

wherein R' and R'' are alkyl or aryl or when taken together with N form a heterocyclic moiety which optionally can contain other hetero atoms on the ring, or acyloxy, and Z is a leaving group or a group that can be converted to a leaving group. Within that aspect of the invention, the presently preferred embodiment is characterized by use of an organometallic compound of the formula ArMX, preferably a magnesium Grignard reagent, and an acyl halide wherein Z is halogen or a group of the formula:

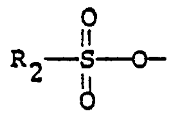

wherein $R_2$ is alkyl, cycloalkyl, alkenyl, alkynyl, aryl or aryalkyl. When Ar is 6-methoxy-2-naphthyl, the utilization of an optically active substituted acyl halide is desirable.

The optically active compound of formula III is preferably then used to prepare an optically active α-aryl alkanoic acid of the formula

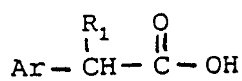

or a pharmaceutically acceptable salt, ester, ortho ester or amide thereof; in which:

(A) the compound of formula III is ketalized to form the corresponding optically active aryl alkyl ketal; and the ketal is rearranged to form a said α-aryl alkanoic acid, or an ester, ortho ester or amide thereof; or

(B) the compound of formula III is contacted with a ketalizing agent to form a first optically active ketal of the formula

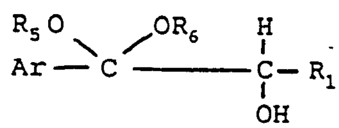

wherein $R_5$ and $R_6$ are alkyl, aryl or aralkyl, optionally the same or different, or when taken together are alkylene having 2 to 8 carbon atoms; a leaving group is regenerated at the α-position of the first ketal to form a second optically active ketal of the formula

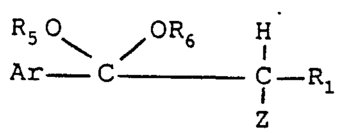

the second ketal is rearranged to form a said α-aryl alkanoic acid, or an ester, ortho ester or amide thereof; or

(C) the optically active compound of formula III is heated with a ketalizing agent in the presence of an acid at an elevated temperature for a time sufficient to afford a said α-aryl alkanoic acid; and optionally

(D) any said ester, ortho ester or amide of route (A) or route (B) is hydrolyzed to the corresponding acid, or

(E) said acid of steps (A) to (D) is converted to a pharmaceutically acceptable salt thereof.

In still another aspect, the invention is directed to a process for producing an optically active α-aryl alkanoic acid of formula

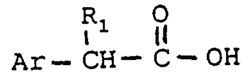

or a pharmaceutically acceptable salt, ester, ortho ester or amide thereof, wherein $R_1$ is alkyl or cycloalkyl and Ar is aryl, from an optically active compound of the formula

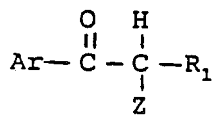

(III)

wherein Z is a leaving group or a group convertible into a leaving group; which comprises:

(A) ketalizing an optically active compound of formula III to form the corresponding optically active aryl alkyl ketal; and rearranging the ketal to form a said α-aryl alkanoic acid, or an ester, ortho ester or amide thereof; or

(B) contacting an optically active compound of formula III with a ketalizing agent to form a first ketal of the formula

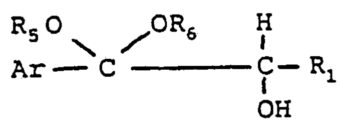

wherein $R_5$ and $R_6$ are alkyl, aryl or aralkyl, optionally the same or different, or when taken together are alkylene having 2 to 8 carbon atoms; regenerating a leaving group at the α-position of the first ketal to form a second optically active ketal of the formula

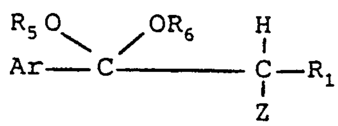

and rearranging the second ketal to form a said α-aryl alkanoic acid, or an ester, ortho ester or amide thereof with the proviso that when $R_5$ and $R_6$ are alkyl Z in the second ketal is other than sulfonyloxy; or

(C) heating an optically active compound of formula III with a ketalizing agent in the presence of an acid at an elevated temperature for a time sufficient to afford a said α-aryl alkanoic acid; and optionally

(D) hydrolyzing any said ester, ortho ester or amide of route (A) or route (B) to the corresponding acid, and further optionally

(E) converting said α-aryl alkanoic acid of steps (A) to (D) to a pharmaceutically acceptable salt thereof.

In another aspect, the invention is directed to a process for producing an optically active ketal of an optically active compound of the formula

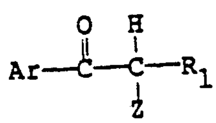

(III)

wherein Ar is aryl, $R_1$ is alkyl or cycloalkyl, and Z is a leaving group or a group convertible to a leaving group with the proviso that when Z is halogen or sulfonyloxy, then the ketal is other than a dialkyl ketal in which Z is hydroxy or sulfonyloxy; which comprises contacting the compound of formula III of which at least a major amount has a preselected absolute configuration with a ketalizing agent effective to maintain said preselected absolute configuration.

That aspect of the invention is particularly characterised by conducting the ketalization with a trialkyl ortho ester such as trimethyl orthoformate or a polyhydric alcohol such as ethylene glycol in the presence of an acid catalyst.

In yet another aspect, the invention is directed to a material comprising a single stereoisomer of a cyclic ketal of a compound of the formula

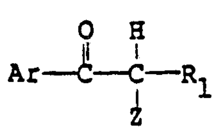

(III)

in the substantial absence of any other stereoisomer of said compound, wherein Ar is substituted or unsubstituted 6-methoxy-2-naphthyl, $R_1$ is alkyl or cycloalkyl, and Z is a leaving group or a group convertible to a leaving group selected from the class of halogen, acetoxy, benzoyloxy, dihydropyranyloxy or trialkylsiloxy.

In yet another aspect, the invention is directed to a material comprising a single stereoisomer of a compound of the formula

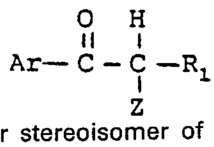 (III)

in the substantial absence of any other stereoisomer of said compound, wherein Ar is 6-methoxy-2-naphthyl, $R_1$ is alkyl or cycloalkyl and Z is a leaving group or a group convertible to a leaving group selected from hydroxy, acetoxy, benzoyloxy, dihydropyranyloxy, trialkylsiloxy or a group of the formula:

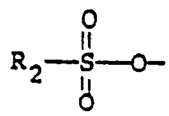

wherein $R_2$ is cycloalkyl, alkenyl or alkynyl.

In still another aspect, the present invention is directed to a material comprising a single stereoisomer of a compound of the formula

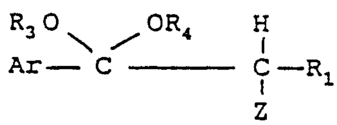

in the substantial absence of any other stereoisomer of said compound, wherein Ar is 6-methoxy-2-naphthyl, $R_1$is alkyl or cycloalkyl, $R_3$ and $R_4$ are alkyl, optionally the same or different, or when taken together, are alkylene having 2—8 carbon atoms, and Z is a leaving group or a group convertible to a leaving group selected from the class of halogen, acetoxy, benzoyloxy, dihydropyranyloxy or trialkylsiloxy.

In still another aspect, the invention is directed to optically active cyclic ketals having an absolute (S)-configuration of the formula:

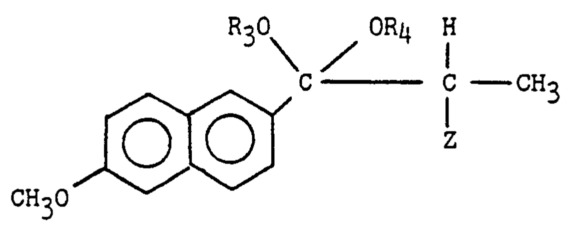

wherein $R_3$ and $R_4$ together provide an alkylene having 2—8 carbon atoms, and Z is halogen, acetoxy, benzoyloxy, dihydropyranyloxy or trialkylsiloxy.

## DETAILED DESCRIPTION OF THE INVENTION

The process of this invention utilizes as starting materials organometallic compounds of the formula:

$$ArMX, (Ar)_2M \text{ or } ArM' \qquad (I)$$

wherein Ar is an aryl moiety, M is cadmium, copper(II), managanese, magnesium or zinc, M' is copper (I) or lithium, and X is a halogen atom. Other starting materials useful in the present invention are substituted acyl halides, acyl amines or acid anhydrides, which may be racemic compounds or optically active compounds, of the general formula:

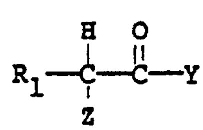 (II)

wherein $R_1$ is alkyl or cycloalkyl, Y is halogen, a group of the formula:

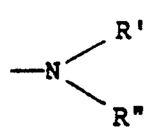

wherein R′ and R″ are alkyl or aryl or when taken together with N form a heterocyclic moiety which optionally can contain other hetero atoms in the ring, or acyloxy, and Z is a leaving group or a group that can be converted to a leaving group. Presently preferred leaving groups are those in which Z is halogen or a group of the formula:

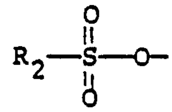

wherein $R_2$ is alkyl, cycloalkyl, alkenyl, alkynyl, aryl or aralkyl.

For the purposes of this invention, alkyl includes straight or branched chain aliphatic groups having 1—18 carbon atoms as exemplified by methyl, ethyl, propyl, isopropyl, n-butyl, sec-butyl, tert-butyl, amyl, hexyl, heptyl, octyl, decyl, dodecyl and octadecyl. Those alkyl groups having 1—8 carbon atoms, and especially those having 1—4 carbon atoms, are presently preferred. Alkenyl groups include those having 2—8 carbon atoms, both straight and branched chain, as exemplified by vinyl, allyl, methallyl, butenyl, pentenyl, hexenyl, heptenyl, octenyl, and isomeric forms thereof. The alkynyl groups include those having 2—8 carbon atoms, both straight chain and branched, as exemplified by ethynyl, propynyl, butynyl, pentynyl, hexynyl, heptynyl, octynyl and isomeric forms thereof. Cycloalkyl include those having 3—15 carbon atoms as exemplified by cyclopropyl, cyclobutyl, cyclopentyl, cyclohexyl, 1-cyclohexenyl, methylcyclohexenyl cycloheptyl, cyclooctyl, cyclodecyl, cycloundecyl, cyclododecyl and cyclopentadecyl. The cycloalkyl groups having 3—8 carbon atoms are presently preferred. The aryl and aralkyl groups comprehended are aromatic groups generally as exemplified by tolyl, xylyl, benzyl, phenethyl, phenylpropyl and benzhydryl. They and the cycloalkyl groups include also fused and bridged ring structures, such as d-10-camphoryl, indanyl, indenyl, naphthyl, napthylmethyl, acenaphthyl, phenanthyl, cyclopentanopolyhydrophenanthyl, adamantanyl, bicyclo[3:1:1]heptyl, bicyclo[2:2:2]octyl and the like. All of the above can either be unsubstituted or substituted with one or more non-interfering subtituents, such as hydroxy or hydroxy derivatives; alkoxy such as methoxy, ethoxy, propoxy, butoxy, and the like; acyloxy, such as acetoxy, propionoxy, butyroxy and the like; nitro groups; alkylamino groups such as dimethylamino and the like; halogens, such as fluorine, chlorine, iodine or bromine; carbonyl derivatives such as enol ethers and ketals; and the like.

The acyloxy groups include those derived from saturated and unsaturated carboxylic acids, carbocyclic carboxylic acids and heterocyclic carboxylic acids. They include, by way of example, straight or branched chain aliphatic groups having 1—18 carbon atoms such as acetoxy, propionyloxy, butyryloxy, isobutyryloxy, palmitoyloxy, stearoyloxy and the like. Also included are the unsaturated aliphatic groups such as acryloyloxy, propioloyloxy, crotonoyloxy, oleoyloxy and the like. Examples of those group derived from the carbocyclic carboxylic acids are benzoyloxy, 2-naphthoyloxy, toluoyloxy, cinnamoyloxy and the like. Examples of those groups derived from heterocyclic carboxylic acids are 3-furoyloxy, 2-thenoyloxy, nicotinoyloxy, isonicotinoyloxy and the like. The acyloxy groups optionally can be substituted with non-interfering substituents, which may include substituents represented by Z as defined herein.

The heterocyclic moieties formed by the group

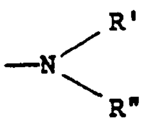

when R′ and R″ are taken together with N, include 5—6 membered ring structures where N is a ring member. Those groups are exemplified by 1- pyrrolyl, 1-imidazolyl, 1-pyrazolyl, 1-piperidyl and the like, or fused ring compounds such as 1-indolyl, 1-H-indazol-1-yl, 3-H-indol-1-yl and the like. The heterocyclic moieties may optionally include other hetero atoms such as oxygen and sulfur. Included therein are morpholino and thiazolyl groups.

The aryl moieties comprehended by Ar include aryl and aralkyl groups generally as described above. The aryl groups include carbocyclic radicals having from 6 to 20 carbon atoms. The carbocyclic radicals may be monocyclic as represented by the phenyl radical or they may be condensed having at least two rings with at least two carbon atoms in common. Examples of condensed aryl radicals are the naphthyl, the indenyl, the anthryl, the acenaptyl, the indanyl and the biphenyl radicals and the like. The carbocyclic radicals can carry additional substituents, for example 1 to 3 lower alkyl radicals and/or lower alkoxy radicals and/or an alkanoyl radical with up to 12 carbon atoms and/or 1 to 3 halogen atoms, more particularly 1 to 3 fluorine, chlorine or bromine atoms and/or an aroyl radical with up to 12 carbon atoms and/or a nitro group. In addition the carbocyclic radicals can also comprise saturated or unsaturated isocylic rings. Examples of unsaturated isocyclic radicals are the phenyl, cyclohexenyl, cyclopentenyl and the naphthyl radicals. Examples of the saturated isocyclic radicals are the cyclohexyl, cyclopentyl, cycloheptyl and the cyclopropyl radicals. Additionally, the carbocyclic radicals may be linked to one or more (up to 4) rings directly by simple bonds to form a ring assembly in accordance with IUPAC Rule A—51. Such a ring assembly may include 5 to 26 carbon atoms, including the carbon atoms of the substituents. Examples of such carbocyclic ring structures include the 4-cyclohexylphenyl, the 4-biphenyl,

the 3-biphenylyl, the 5-cyclohexyl-1-indanyl, the 4-(1-cyclohexen-1-yl)phenyl and the 5-phenyl-1-naphthyl radicals and the like. Those ring assemblies can carry from 1 to 3 substituents such as described above. In particular, the aryl moieties comprehended include those of acid products such as exemplified in U.S. Patents Nos. 3,385,386; 3,660,437; 3,624,142; 3,755,427; 3,904,682; and 3,912,748 and Belgian Patent No. 747,812. Described therein are substituted or unsubstituted phenyl, phenoxyphenyl, naphthyl or biphenyl groups, such as represented by 3-phenoxyphenyl, 2-fluoro-1,1'-biphenylyl, 4-isobutylphenyl, 4'-fluoro-4-biphenylyl, 6-methoxy-2-naphthyl, 5-bromo-6-methoxy-2-naphthyl and 4-chlorophenyl-5-benzoxazoyl.

The halogens comprehended herein are bromine, chlorine, fluorine and iodine, with bromine and chlorine being presently preferred.

Z may itself be a leaving group or a group which is convertible to a leaving group. Leaving groups include the anions of inorganic and organic acids.

Those groups are sufficiently labile to disassociate from the substrate upon and/or by contacting of the substrate with Lewis acid, an agent having an affinity for oxygen or a protic or dipolar aprotic solvent during rearrangement of the aryl α-substituted alkyl ketal to the α-arylalkanoic acid or ester, ortho ester or amide thereof. Typical leaving groups are the halogens as exemplified by bromine, chlorine and iodine. Alternatively, Z is an anionic residue of an organic acid. Particularly suitable organic acids are those having electron deficient substituents such as exemplified by aryl, aralkyl, cycloalkyl, alkyl, alkenyl and alkynyl sulfonic acids and substituted benzoic and phosphonic acids. Presently preferred are those leaving groups wherein Z represents a group of the formula:

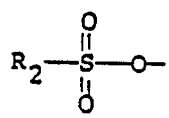

wherein $R_2$ is alkyl, cycloalkyl, alkenyl, alkynyl, aryl or aralkyl. Among such leaving groups, those in which $R_2$ is alkyl, aryl or aralkyl are especially advantageous, such as methanesulfonyloxy, benzenesulfonyloxy and p-toluenesulfonyloxy.

Groups represented by Z which can be converted to leaving groups are exemplified by hydroxy and by protected hydroxy groups such as acetoxy, benzoyloxy, trialkylsiloxy (e.g. trimethylsiloxy and triethylsiloxy) and dihydropyranyloxy. In some instances, the group represented by Z may itself be a leaving group which can be converted into a different leaving group at a subsequent stage of the process. All such variations are comprehended by Z in its broadest representation.

The organometallic reagents of formula I are conveniently prepared by conventional methods for preparing Grignard reagents. The appropriate aryl halide of the formula:

Ar—X

is dissolved in an anhydrous, aprotic medium such as an ether, for example, tetrahydrofuran, diethyl ether and the like, or mixtures thereof, and added to magnesium metal. Preparative methods analogous to those described in U.S. Patent No. 3,959,364 can be utilized. For example, 2-bromo-6-methoxynaphthalene is dissolved in an ether such as tetrahydrofuran and the resulting solution added slowly to magnesium to form the magnesium Grignard of 2-bromo-6-methoxynaphthalene. The other divalent metallic derivatives, i.e. of cadmium, copper(II), manganese and zinc, are prepared from the magnesium Grignard of the aryl halide, Ar-X, by conventional exchange procedures, such as have been described in U.S. Patents Nos. 3,658,858 and 3,975,432. Typically, the magnesium Grignard of the aryl halide is contacted with halide of the metal to be exchanged in a suitable solvent, such as a hydrocarbon solvent, at elevated temperatures. Whether the compound of the formula ArMX or $(Ar)_2M$ is formed depends on the amount of metal halide employed in the reaction. The compound of the formula ArMX is primarily formed when one molar equivalent of the metal halide is employed, and the compound of the formula $(Ar)_2M$ primarily is formed when one-half molar equivalent of the metal halide is employed. For example, the contacting of one molar equivalent of 2-bromo-6-methoxynaphthalene with one-half molar equivalent of zinc chloride in benzene yields a solution of di-(6-methoxy-2-naphthyl)zinc. When one molar equivalent of zinc chloride is employed, a solution of (6-methoxy-2-naphthyl)zinc chloride is obtained. The organo lithium compounds are prepared directly from the aryl halide by contacting the aryl halide with lithium metal in a manner similar to the preparation of the magnesium Grignard. The copper(I) compound is prepared directly from the lithium derivative by reaction with cuprous bromide in an ether solvent (U.S. Patent No. 3,658,863).

The substituted acyl halides of formula II are prepared from corresponding acids by α-halogenation of the alkanoic acid by the addition of halide in the presence of a catalytic amount of phosphorus trichloride (Hell-Volhard-Zelinsky reaction) to afford the α-haloalkanoic acid. In the case where Z is halogen, the α-haloalkanoic acid is converted directly to the desired acyl halide by reaction with thionyl chloride, phosgene, phosphorous pentachloride or the like. While other acyl halides, e.g. bromides and iodides, can be utilized, the acyl chlorides are generally satisfactory for subsequent additional to the organometallic reagent.

When Z comprises an ester leaving group such as exemplified by a group of the formula:

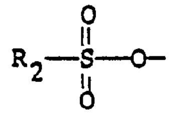

or another anionic residue of an organic acid, the α-haloalkanoic acid is hydrolyzed to the α-hydroxyalkanoic acid, esterified to form the α-hydroxyalkanoate and treated with an appropriate organic acid halide to form the diester. Subsequent hydrolysis affords the α-subtituted alkanoic acid. At this stage, the α-substituent does not hydrolyze since the terminal ester group hydrolyzes much more rapidly than the α-substituent. Then the α-substituted alkanoic acid is treated with a halide such as thionyl chloride, benzenesulfonyl chloride, phosgene, phosphorus trichloride or pentachloride or the like to afford the α-substituted acyl halide, which typically is an α-substituted acyl chloride. That sequence of reactions is illustrated by the addition of bromine to propionic acid in the presence of phosphorus trichloride to afford α-brompropionic acid. The α-bromopropionic acid is hydrolyzed with base such as potassium hydroxide, to form the α-hydroxypropionic acid which is esterified with an alcohol, e.g. ethanol, under acidic conditions to afford ethyl 2-hydroxypropionate (i.e. ethyl α-hyddyroxypropionate). Contacting of ethyl 2-hydroxypropionate with methanesulfonyl chloride yields ethyl 2-methanesulfonyloxypropionate, which is hydrolyzed with potassium hydroxide to afford 2-methanesulfonyloxypropionic acid. Further reaction of that material with thionyl chloride yields 2-methanesulfonyloxypropionyl chloride, which is subsequently utilized.

The optically active α-substituted acyl halides are prepared by resolution of the racemic α-hydroxyalkanoic acids or esters by conventional methods using optically active amine bases or from available amino acids, for example, such as by methods described in the *Journal of the American Chemical Society, 76,* 6054 (1954).

When α-arylpropionic acids are to be prepared by the process of this invention, a particularly advantageous starting material is lactic acid (i.e. 2-hydroxypropionic acid). The naturally occurring lactic acid, L-(+) lactic acid, is optically active and as such is a preferred starting material for the stereoselective processes described herein. Alternatively, the ethyl ester of L-(+)lactic acid also is commercially available (Pettibone World Trade, Chicago, Illinois and C.V. Chemie Combinatie, Amsterdam C.A.A., Holland) and is a convenient starting material for the optically active α-substituted propionyl halides utilized in the stereoselective process of this invention. Depending on the number of inversions at the asymmetric carbon atom of the propionic acid group during subsequent steps in the process, as will be described more fully hereinafter, either the (+)-lactic acid or the (−)-lactic acid is the preferred starting material. The (−)-lactic acid can be obtained from the racemic lactic by conventional resolution methods or prepared directly from glucose via a method described in Biochemical Prepn., *3,* 61 (1953).

The optically active, substituted propionyl halides are prepared from the optically active ester of the appropriate lactic acid enantiomer by treating that enantiomer with an organic acid halide, such as, for example, sulfonic acid halides of the formula:

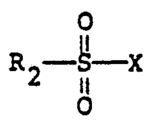

wherein $R_2$ and X are as defined above, to form the α-substituted propionate. Basic hydrolysis of the ester to the acid, such as with potassium hydroxide in aqueous methanol, and subsequent treatment with a halogenating agent, such as thionyl chloride, yields the optically active α-substituted propionyl halide. Typically, (S) ethyl 2-hydroxypropionate, corresponding to the ethyl ester of L-(+)lactic acid, is treated with methanesulfonyl chloride, in the presence of an organic base, such as triethylamine, and an inert solvent, such as toluene, to afford (S) ethyl 2-methanesulfonyloxypropionate. Basic hydrolysis of that material with potassium hydroxide in aqueous methanol yields (S) 2-methanesulfonyloxypropionic acid, which then is allowed to react with thionyl chloride to afford (S) 2-methanesulfonyloxypropionyl chloride.

In order to form the (R) substituted propionyl chloride, one begins with (R) ethyl 2-hydroxypropionate and proceeds through the above-described process sequence to obtain the (R) 2-substituted propionyl halide such as (R) 2-methanesulfonyloxypropionyl chloride.

The acyl amines represented by formula II when Y is a group of the formula:

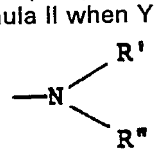

wherein R′ and R″ are alkyl or aryl or when taken together with N form a heterocyclic moiety which optionally can contain other hetero atoms, are prepared from the acyl halides and an N,N-disubstituted amine or the parent nitrogen containing heterocycle. For example 2-methanesulfonyloxypropionyl chloride is contacted with dimethylamine to afford N,N-dimethyl 2-methanesulfonyloxypropionamide. Other

disubstituted amines can be utilized as well. The acryl amines derived from heterocyclic amines having substantial acid character such as the imidazoles, pyrroles, indoles and carbazoles are also consdidered useful.

The acyl halides can be converted to the symmetrical or mixed acid anhydrides corresponding to the compounds of formula II when Y is acyloxy, by contacting the acyl halide with an appropriate acid. For example, 2-methanesulfonyloxypropionyl chloride is allowed to react with acetic acid to afford the mixed anhydride, acetic 2-methanesulfonyloxypropionic anhydride. Additionally, for example, 2-methanesulfonyloxypropionyl chloride is allowed to react with 2-methanesulfonylotypropionic acid to afford bis(2-methanesulfonyloxypropionic)anhydride, a symmetrical anhydride. Alternatively, the acid precursors of the acyl halides of formula II can be contacted with appropriate acyl halide to afford the symmetrical or mixed acid anhydrides. For example, 2-methanesulfonyloxypropionic acid is allowed to react with acetyl chloride to afford the mixed anhydride, acetic 2-methanesulfonyloxypropionic anhydride.

The optically active acyl amines and acid anhydrides are prepared conveniently from the optically active acyl halides in the manner described above to yield materials particularly useful in the stereospecific process of this invention.

The compounds represented by formula II also encompass compounds in which Z is a group which can be converted into a suitable leaving group at subsequent stages of the process, i.e. at some point after the compound of formula I has been allowed to react with the compound of formula II. Such groups are, for example, hydroxy, and protected hydroxy groups such as acetoxy, benzoyloxy, dihydropyranyloxy, trialkylsiloxy and the like. Typically, the α-hydroxy substituent of the α-hydroxyalkanoic acid or an ester thereof is protected during the addition of the compound of formula II to the compound of formula I. Subsequently, the protecting group is removed and a suitable leaving group is generated. Typically, (S) 2-hydroxypropionic acid is treated with acetyl chloride in the presence of sulfuric acid to yield (S) 2-acetoxypropionic acid. Subsequent treatment with thionyl chloride affords (S) 2-acetoxypropionyl chloride, a compound of formula II.

The compound of formula I is allowed to react with the compound of formula II to yield a compound of the formula:

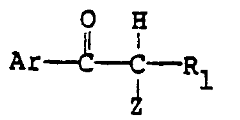

(III)

wherein Ar, $R_1$, and Z are as defined above. By employing an optically active acyl halide, acyl amine or acid anhydride of the formula II, the corresponding optically active aryl α-substituted alkyl ketone is obtained. That reaction step proceeds with substantially 100% retention of optical activity. For example, the magnesium Grignard of 2-bromo-6-methoxynaphthalene is contacted with (S) 2-methanesulfonyloxypropionyl chloride in an inert solvent such as tetrahydrofuran to yield (S) 1 - (6 - methoxy - 2 - naphthyl) - 2 - methanesulfonyloxypropan - 1 - one. The use of the (R) form of the acyl halide produces the (R) form of the ketone, e.g. (R) 1 - (6 - methoxy - 2 - naphthyl) - 2 - methanesulfonyloxypropan - 1 - one. Reaction conditions for this step of the process are not considered critical. Generally, the reaction is conducted at temperatures below room temperature. For example, the range of −70°C to °C is suitable and a range of −60°C to −20°C is presently preferred. Inert solvents such as the ethers (e.g. tetrahydrofuran) form a convenient medium for conducting the reaction. The solvents may be used alone or as mixtures. The ratio of the acyl halide, acyl amine or acid anhydride to the organmetallic compound typically is between 1.0—1.5 equivalents, although greater excesses can can be used.

The racemic and optically active ketones of the formula:

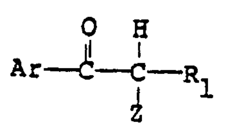

(III)

can be utilized in alternate processes to yield the desired α-arylalkanoic acids. Representative process schemes are illustrated below.

REACTION SCHEME I

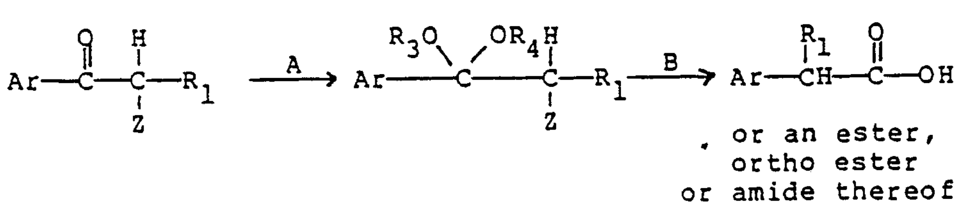

In Scheme I, Ar, $R_1$ and Z are as defined above. $R_3$ and $R_4$ are alkyl having 1—8 carbon atoms, optionally the same or different, or when taken together, are alkylene having 2—8 carbon atoms.

In Reaction Scheme I, the ketalization step (step A) is conducted under conditions of retention of configuration at the asymmetric carbon atom. Typically, the ketal is formed by contacting the ketone with an ortho ester in the presence of an acid catalyst in an alcoholic solvent. Such a method utilizes trialkyl orthoformates, such as trimethyl orthoformate or triethyl orthoformate in the presence of an acid catalyst such as sulfuric acid, p-toluenesulfonic acid, ferric chloride, ammonium nitrate, ammonium chloride or acidic ion exchange resins such as Amberlyst-15, Nafion H(perfluoronated sulfonate polymer) and acidic montmorillonite clay (e.g. Girdler® catalyst K—10, Girdler Chemicals Inc., Louisville, Kentucky). Typically, (S)1 - (6 - methoxy - 2 - naphthyl) - 2 - methanesulfonyloxypropan - 1 - one is contacted with trimethyl orthoformate in the presence of sulfuric acid to yield (S) 1,1 - dimethoxy - 1 - (6 - methoxy - 2 - naphthyl)prop - 2 - ylmethanesulfonate.

Cyclic ketals are formed by using glycols and other polyhydric alcohols such as ethylene glycol, trimethylene glycol 1,3-butanediol, 1,4-butanediol, 1,4-pentanediol, 1,5-pentanediol, 2,2-dimethyl-1,3-propanediol (neopentyl glycol), 1,5-hexanediol, 1,6-hexanediol, 2,5-hexanediol, 1,8-octanediol, and the like in the presence of an acid catalyst. The water which forms is removed by azeotropic distillation. Other useful ketalization agents are trimethylsilyl trifluoromethanesulfonate with an alkoxysilane [Tetetrahedron Letters, 21, 1357—8 (1980)], 2,2-dimethoxypropane and dimethylsulfite. The ketalization agents can be used in amounts of about 1—50 molar equivalents per molar equivalent of ketone. When the orthoformates are utilized, a range of 2.0—5.0 equivalents is satisfactory. Usually about 0.5—20 percent on a molar basis of acid catalyst is utilized. Inert solvents such as alcohols, benzene, toluene and the like are utilized. When the cyclic ketals are formed from the polyhydric alcohols, a solvent such as toluene or benzene typically is used to allow azeotropic distillation of the water formed. While temperatures are not critical, temperatures in the range of 0°C to 150°C are typical depending on the solvent and the ketal.

When trimethyl orthoformate is utilized as the ketalization agent, the addition of an inert co-solvent such as toluene or methylene chloride appears to solubilize the ketone and lead to reduced usage of trimethyl orthoformate. A temperature range of 35°C to 80°C is presently preferred and about 30 percent on a molar basis of oleum gives particularly satisfactory results. Reaction times of hours to days are considered typical depending on the nature of the solvent and the temperature and the amount of ketalizing agent used.

The rearrangement step (step B) can be conducted by a variety of methods depending to some extent on the nature of the leaving group Z.

When Z is halogen, the rearrangement is conveniently conducted in an inert solvent in the presence of catalyst such as a Lewis acid (see for example European Patent Office Application No. 81200210.3, filed February 23, 1981, bearing publication No. 0034871, published September 2, 1981). Representative catalysts are the organic salts, such as acetate, propionate, benzoate, trifluoromethanesulfonate, methanesulfonate, and the like, and the inorganic salts such as chloride, bromide, iodide, sulfate and the like of copper, magnesium, calcium, zinc, cadmium, barium, mercury, tin, antimony, bismuth, manganese, iron, cobalt, nickel and palladium. The metal halides such as zinc chloride, cobalt chloride, zinc bromide, stannous chloride, ferrous chloride, ferric chloride, nickel bromide, cadmium chloride, magnesium chloride, mercurous chloride, mercuric chloride, antimony chloride, barium chloride, calcium chloride, cuprous chloride, cupric chloride, manganese chloride, stannic chloride, bismuth chloride and palladium trichloride are considered particularly useful. The rearrangement is conducted in a suitable solvent such as the aliphatic halohydrocarbons, aliphatic cyclic hydrocarbons, lower alcohols, aliphatic acids and esters thereof, aromatic hydrocarbons and haloaromatic hydrocarbons. Representative examples are dichloromethane, trichloromethane, chlorobenzene, toluene, methylene chloride, methanol, trimethyl orthoformate and mixtures thereof. The rearrangement is conducted in the temperature range from about 0°C to the reflux temperature of the solvent, with due consideration given to the temperature stability of the ketal and the resulting acid or esters thereof. Reaction times are not critical and vary with the nature of the ketal and the catalyst and the reaction temperature. Times ranging from about 0.5 hours to 160 hours are considered representative. Other particulars of the rearrangement process can be found in the above-referenced European Patent Application, Publication No. 0034871, which is incorporated herein by reference.

An alternative rearrangement process when Z is halogen has been described in British Application No. 8005752 filed February 20, 1980, bearing publication No. 2,042,543, published September 24, 1980, and which is incorporated herein by reference. That process utilizes the silver (I) salts of organic or inorganic anions as catalysts for the rearrangement step in an acidic, alcoholic medium. The acid typically is selected from the Lewis acids, boron trifluoride, fluoroboric acid, methanesulfonic acid, sulfuric acid, the complexes $BF_3.2CH_3COOH$, $HBF_4$ $.Et_2O$ (etherated fluoroboric acid), $BF_3.Et_2O$ (boron trifluoride etherate) and $BF_3.2CH_3OH$. The silver salts are the silver (I) salts of organic and/or inorganic anions, mixtures thereof, and silver oxide. Representative silver salts are silver acetate, $AgSbF_6$ (silver hexafluoroantimoniate), $AgClO_4$ (silver perchlorate), $AgCF_3SO_3$ (silver trifluoromethane sulfonate), $AgBF_4$ (silver tetrafluoroborate), silver nitrate, silver carbonate, silver sulfate and silver oxide. The rearrangement is conducted in a protic or dipolar, aprotic solvent, such as provided by an alcoholic medium, including the alkanols (e.g. methanol,

ethanol), and cycloalkanols, and as provided by the orthoformates, acetone dimethylacetal or the $BF_3.2CH_3OH$ complex.

When Z comprises an ester leaving group of the formula:

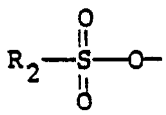

or another anionic residue of an organic acid, the ketal formed in step A is solvolyzed according to step B to the rearranged α-arylalkanoic acid or an ester, ortho ester or amide thereof, depending on the solvolysis conditions. The solvolysis is conducted under either basic, neutral or acidic conditions. Reaction times, temperatures and material ratios during the solvolysis step are not considered critical. Typically, temperatures in the range between 0°C and 200°C and times ranging between 1—100 hours are satisfactory. Temperatures of greater than 50°C appear to accelerate the rearrangement. Typically, the solvolysis is effected by maintaining the ketal in contact with a protic or dipolar, aprotic solvent for a time sufficient to form α-arylalkanoic acid or the ester, ortho ester or amide thereof. Protic solvents comprehended include water, alcohols, ammonia, amides, N-alkylamides, carboxylic acids and mixtures thereof.

Representative alcohols include primary, secondary and tertiary alcohols and polyhydric alcohols. They include alkanols, alkenols, cyclic alkanols, aromatic alcohols, glycols, and the like. Examples of the alkanols comprehended are methanol, ethanol, butanol, pentanol, hexanol, heptanol, octanol, and the branched chain isomers thereof. Examples of the alkenols are allyl alcohol, 2-buten-1-ol and the like. Cyclic alkanols are exemplified by cyclopropanol, cyclobutanol, cyclohexanol and the like. Examples of aromatic alcohols are phenol, α-naphthol, β-naphthol, p-cresol and the like. Representative amides are formamide, acetamide, propionamide, benzamide and the like. Typical of the N-alkyl amides are N-methylformamide and N-ethylformamide. Carboxylic acids are alkanoic acids such as formic acid, acetic acid, propionic acid, n-butyric acid and the branched chain isomers thereof; alkenoic acids, such as acrylic acid, maleic acid and fumaric acid and the like; aryl acids such as benzoic acid, and the like, and diacids such as phthalic acid, isophthalic acid, malonic acid, succinic acid, glutaric acid and the like.

Dipolar, aprotic solvents are typified by dimethylsulfide, acetone, dioxane, 1,2-dimethoxyethane, carbon disulfide, dialkylamides such as dimethylacetamide and dimethylformamide, nitrobenzene, nitromethane, acetonitrile and the like and mixtures thereof.

The rate of the rearrangement reaction appears to be enhanced by the presence of salts of organic or inorganic anions. For example, the addition of sodium acetate or sodium bicarbonate to the reaction mixture facilitates the reaction. Additionally, it is sometimes desirable to buffer the solvent medium to prevent hydrolysis of the ketal prior to occurance of the rearrangement. Typical buffering salts include the calcium, sodium, potassium and lithium salts of carbonate, bicarbonate, anions of organic acids and phosphates.

Depending on the nature of the protic or dipolar aprotic solvent medium, the α-arylalkanoic acid may not be directly formed. Instead, the ester, ortho ester or aide of the α-arylalkanoic acid may be formed. For example, if the solvent medium contains water, an ester of the α-arylalkanoic acid tyically is formed wherein the ester group is derived from the ketal functionality or from the solvent. Mixed esters can be formed. Under anhydrous alcoholic conditions, ortho esters of the α-arylalkanoic acid can be formed wherein the ester groups may be derived from the ketal functionality or from the solvent and may be mixed. Likewise, when an amine is present in the solvent medium, formation of an amide of the α-arylalkanoic acid can be expected. Those compounds typically are not isolated but are hydrolyzed directly to the desired α-arylalkanoic acid.

Depending on the reaction conditions, hydrolysis of an ester, ortho ester or amide formed may be effected concomitantly or sequentially by standard methods. For example, when the protic solvent medium comprises acetic acid and sodium acetate and the ester substrate comprises 1,1 - dimethoxy - 1 - (6 - methoxy - 2 - naphthyl)prop - 2 - yl methanesulfonate, there is afforded the compound, methyl 2-(6-methoxy-2-naphthyl)propionate. The methyl ester is then hydrolyzed to the corresponding acid by contact with base. Alternatively, the α-arylalkanoic acid can be obtained by concomitant hydrolysis by maintaining the ester substrate in contact with a methanol-water solution containing sodium bicarbonate. Typically, 1,1 - dimethoxy - 1 - (6 - methoxy - 2 - naphthyl)prop - 2 - yl methanesulfonate is maintained in contact with a methanol-water solution containing sodium bicarbonate to afford 2 - (6 - methoxy - 2 - nahthyl)propionic acid.

When the stereoselective process described herein is utilized to produce the optically active esters of the α-arylalkanoic acids, it has been determined that the presence of excess base during the solvolytic rearrangement step can racemize the optically active ester so produced. Accordingly, it is presently desirable to minimize the amount of base which is in contact with the optically active ester. That can be accomplished by conducting the solvolysis under buffered acidic conditions or in the presence of an insoluble base, i.e. a base which is insoluble in the solvent phase containing the optically active ester, or in a weakly basic media. For example, when the solvolysis is conducted in methanol, the use of calcium carbonate or resin bases as the insoluble base gives satisfactory results. For example, when (S) 1,1 -

dimethoxy - 1 - (6 - methoxy - 2 - naphthyl)prop - 2 - yl methanesulfonate is maintained in contact with an aqueous methanol solution in which calcium carbonate is stirred as an insoluble base, (S) methyl 2-(6-methoxy-2-naphthyl)propionate is obtained in greater than 95 percent optical purity. The methyl ester then is converted to (S) 2-(6-methoxy-2-naphthyl)propionic acid by acid catalysis or saponification, where such saponification is conducted in an aprotic solvent such as acetone.

The problem of isomerization in the presence of excess base appears to be due in part to the relative slowness of the rearrangement step. During solvolysis, at any one time only a small amount of sulfonic acid, e.g. methanesulfonic acid, is being formed. If an insoluble, substantially insoluble, or weak base is utilized, only a very low concentration of hydroxyl ions are present. The low concentration is sufficient to neutralize the acid formed but is insufficient to isomerize the optically active ester formed.

Alternatively, the rearrangement step (step B) can also be effected by contacting the ketal formed in step A with an agent having affinity for oxygen. Under such conditions, the ester of the α-arylalkanoic acid is produced.

The agents having an affinity for oxygen are those compounds having the ability to coordinate to accept a lone electron pair of an oxygen atom. Representative examples are iodotrialkylsilanes, such as iodotrimethylsilane, iodotriethylsilane and the like, the trialkylsilyl perfluoroalkylsulfonates such as trimethylsilyl trifluoromethanesulfate, trimethylsilyl pentafluoroethanesulfonate and the like, and Lewis acids such as aluminum chloride, aluminum bromide, zinc chloride, stannous chloride, stannic chloride, titanium chloride, boron fluoride, ferric chloride, ferrous chloride and the other Lewis acids described previously. The agents having affinity for oxygen can be used alone or as mixtures.

The amount of the agent having an affinity for oxygen used depends to some extent on the type of the ketal being rearranged and/or on the type of the agent. Generally, it is used in an amount of about 0.1 to 5.0 moles per mole of the ketal formed in step A. A range of 1.0—2.0 moles is presently preferred.

The treatment of the ketal formed in step A with the agent having affinity for oxygen can be carried out in the absence of solvent. However, the process is conveniently carried out in a solvent, especially an aprotic solvent. For example, where a Lewis acid or an iodotrialkylsilane is used, halogenated hydrocarbons such as methylene chloride, chloroform and 1,2-dichloroethane are advantageous. When the trialkylsilyl perfluoroalkanesulfonates are used, the halogenated hydrocarbons, acetonitrile and orthoformates are presently preferred as solvents.

Reaction conditions can vary widely, although a temperature range of about −40°C to about 150°C is satisfactory. A range of about −20°C to about 100°C, and especially about −10°C to about 90°C, is presently preferred.

When the agent having affinity for oxygen is a Lewis acid, the product produced in step B may sometimes form a complex with the agent. In this case, the product may be isolated by adding water to the reaction mixture to decompose the complex. The desired product is then isolated by conventional methods, such as extraction, chromatography, distillation and crystallization.

When Z is a group which is convertible to a suitable leaving group, the conversion of Z can take place either before step A or thereafter, but before step B. For example, when the magnesium Grignard of 2-bromo-6-methoxynaphthalene is contacted with (S) 2-trimethylsiloxypropionyl chloride, (S) 1 - (6 - methoxy - 2 - naphthyl) - 2 - trimethylsiloxypropan - 1 - one is obtained. Regeneration of the hydroxyl group by hydrolysis affords (S) 2-hydroxy-1-(6-methoxy-2-napthyl)propan-1-one. Further reaction with methanesulfonyl chloride then affords (S) 1 - (6 - methoxy - 2 - naphthyl) - 2 - methanesulfonyloxypropan-1-one, which is processed according to step A. Alternatively, the ketone can first be ketalized according to step A and the regeneration of the hydroxyl group and the formation of the sulfonat ester can occur subsequently but prior to step B.

When it is desired to practice the stereoselective process described herein, the particular stereoisomer of the material described by the formula:

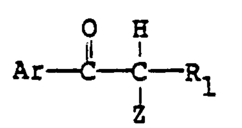

(III)

is produced to provide the desired optically active stereoisomer of the formula:

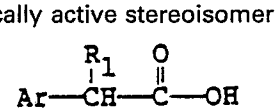

or an ester, ortho ester or amide thereof, wherein Ar, $R_1$ and Z are as defined above. In Reaction Scheme I, the ketalization step A proceeds under conditions of retention of configuration and the rearrangement step B proceeds under conditions of inversion of configuration at the asymmetric carbon atom. Accordingly, for example, when it is desired to produce (S) (6-methoxy-2-naphthyl)propionic acid, the (S) form of ethyl lactate is utilized to produce (S) 2-methanesulfonyloxypropionyl chloride in the manner described previously. Utilization of the (S)-form of that reagent with the magnesium Grignard of 6-methoxy-2-bromonaphthalene affords (S) 1-(6-methoxy-2-naphthyl)-2-methanesulfonyloxypropan-1-one. Ketalization with retention of configuration with trimethyl orthoformate in the presence of a catalytic amount of sulfuric

acid yields (S) 1,1-dimethoxy-1-(6-methoxy-2-naphthylprop-2-yl methanesulfonate. Rearrangement of that material with sodium acetate and acetic acid in ethanol proceeds with inversion of configuration at the asymmetric carbon atom and affords (S) ethyl 2-(6-methoxy-2-naphthyl)propionate. That material is then hydrolyzed to the corresponding (S) acid.

Because the "sequence rule," when assigning an "R" or "S" configuration, depends on the nature of the groups attached to the asymmetric carbon atom, the absolute configuration of the starting and ending material in the rearrangement step is each "S" in the example given, even though there is inversion of configuration at the asymmetric carbon atom. The migration of the 6-methoxy-2-naphthyl group to the asymmetric carbon atom with inversion of configuration at that carbon atom dictates that notation.

When Z is a group of the formula:

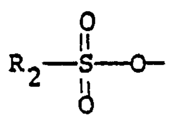

wherein $R_2$ is alkyl, cycloalkyl, alkenyl, alkynyl, aryl or aralkyl, or another anionic residue of an organic acid, step A and step B can be conveniently combined by conducting the ketalization (Step A) at elevated temperatures to effect rearrangement (step B) to the ester of the α-arylalkanoic acid. That combined ketalization-rearrangement typically is conducted at elevated temperatures in excess of 80°C and up to the boiling temperature of the solvent, at appropriate pressures to attain those elevated temperatures with the solvents being utilized. Typically, (S) 1-(6-methoxy-2-naphthyl)-2-methanesulfonyloxypropan-1-one is contacted with trimethyl orthoformate in the presence of 98 percent sulfuric acid in methanol and heated under 50 psi to above 80°C, to afford (S) methyl 2-(6-methoxy-2-naphthyl)propionate. Reaction times, ranging from hours to days, can vary depending on the amount of orthoformate used and the temperature at which the process is conducted. Higher temperatures may require correspondingly higher operating pressures depending on the solvents utilized.

An alternative scheme for the preparation of the α-arylalkanoic acids and esters proceeds with two inversions at the asymmetric carbon atom and is represented by the following reaction sequence:

REACTION SCHEME II

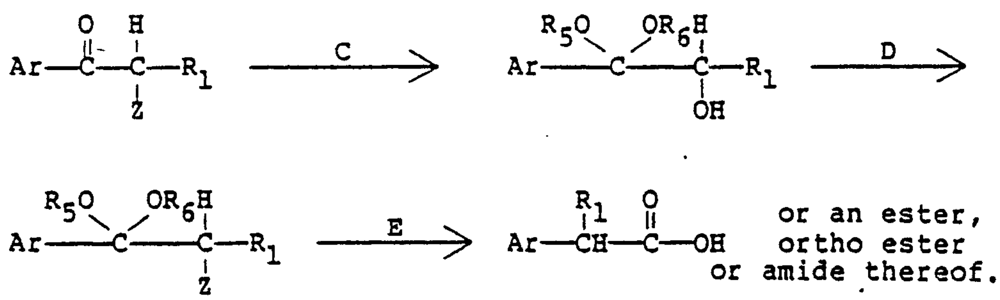


In Scheme II Ar, $R_1$ and Z are as defined above with the understanding that Z prior to step C may be the same or different than Z after step D. $R_5$ and $R_6$ are alkyl, aryl or aralkyl, optionally the same or different, or, when taken together, are alkylene having 2—8 carbon atoms. Scheme II differs from Scheme I in that the ketalization step C takes place under conditions which result in the formation of the α-hydroxy ketal with inversion of configuration at the asymmetric carbon atom. Ketalization with alkali metal alkoxides, aralkyloxides or aryloxides in an alcoholic medium yields the α-hydroxy ketals in which inversion at the asymmetric carbon atom has occurred. Representative alkali metals are sodium, potassium and lithium. The alkoxides contain 1—8 carbon atoms and are exemplified by methoxide, ethoxide and the like. Aralkyloxides and aryloxides are exemplified by benzyloxide, phenoxide and the like. Alternatively, the cyclic ketals can be formed with the polyhydric alcohols described previously in Scheme I in the presence of a catalytic amount of the alkali metal alkoxides to afford the α-hydroxy cyclic ketals in which inversion at the asymmetric carbon atom has occurred.

Regeneration of a leaving group Z in step D typically is effected by contacting the α-hydroxy ketal with an organic acid halide, such as an alkyl, alkenyl, alkynyl, cycloalkyl, aryl or aralkyl sulfonyl halide. That step occurs with retention of configuration. Rearrangement of the ester in step E takes place with inversion of configuration utilizing the methods described previously with respect to Scheme I.

Since Scheme II involves two inversions at the asymmetric carbon atom, (i.e. one inversion during the ketalization step and one inversion during the rearrangement step) the appropriate stereoisomer of the compound of the formula:

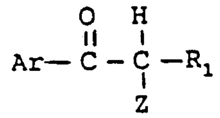

(III)

wherein Ar, $R_1$, and Z are as defined above, must be chosen to provide the desired optically active stereoisomer of the α-arylalkanoic acid being produced. For example, to produce (S) 2-(6-methoxy-2-naphthyl)propionic acid or an ester, ortho ester or amide thereof according to the procedure of Scheme II, it is necessary to begin with (R) ethyl lactate. That material is converted to (R) 2-methanesulfonyloxypropionyl chloride in the manner described herein and allowed to react with the magnesium Grignard of 2-bromo-6-methoxynaphthalene to yield (R) 1-(6-methoxy-2-naphthyl)-2-methanesulfonyloxypropan-1-one. Ketalization in step C with sodium methoxide in methanol then affords, with inversion of configuration, (S) 1,1-dimethoxy-1-(6-methoxy-2-naphthyl)propan-2-ol. Ester formation in step D occurs by contacting the (S)-ketal above with methanesulfonyl chloride to afford (S) 1,1-dimethoxy-1-(6-methoxy-2-naphthyl)prop-2-yl methanesulfonate, which is rearranged with inversion by contacting with sodium acetate and acetic acid to afford (S) methyl 2-(6-methoxy-2-naphthyl)propionate. The foregoing example is again illustrative of the pecularities of the "sequence rule" of nomenclature, which, when utilized to assign absolute configurations to asymmetric carbon atoms, can result in an (S)-compound being converted to another (S)-compound even though inversion at the asymmetric carbon atom has occurred. That apparent inconsistency is in reality no inconsistency at all because the assignment of the "R" or "S" configuration depends on the nature of the groups attached to the asymmetric carbon atom.

The foregoing illustration is representative of the situation wherein the leaving group Z prior to step C is the same as the leaving group Z prior to step E. The leaving group Z in each instance need not be identical. For example, Z prior to step C can be a halogen, such as bromo and chloro, which is eliminated in the formation of the α-hydroxyketal. Then, the α-hydroxyketal can be contacted with methanesulfonylchloride to yield the ketal wherein the leaving group Z is methanesulfonyloxy.

The ketones of the formula III wherein Z is halogen can also be prepared from compounds prepared from (R), (S) or (RS) lactic acid or the esters thereof. In this aspect of the invention, the compounds of formula III wherein Z is a group of the formula:

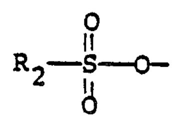

wherein $R_2$ is as previously defined are contacted with an alkali metal halide to yield the optically active or racemic compound of formula III in which Z is halogen. Typically, lithium bromide or lithium chloride is used, or a phase transfer catalyst is employed with potassium bromide in a non-polar organic solvent such as toluene. In certain solvents the reaction proceeds with racemization at the asymmetric carbon atom and as such is not universally suitable for the preparation of the optically active α-halo ketones. For example, (R) 1-(6-methoxy-2-naphthyl)-2-methanesulfonyloxypropan-1-one is contacted with lithium bromide in dimethylformamide to afford substantially all (RS) 2-bromo-1-(6-methoxy-2-naphthyl)propan-1-one. That material is suitable for use in Reaction Scheme I to produce racemic 2-(6-methoxy-2-naphthyl)propionic acid. Similarly, (S) 1-(6-methoxy-2-naphthyl)-2-methanesulfonyloxy-propan-1-one is contacted with lithium bromide to afford (RS) 2-bromo-1-(6-methoxy-2-naphthyl)propan-1-one, which is suitable for use in Reaction Scheme II to produce racemic 2-(6-methoxy-2-naphthyl)propionic acid.

Because application of the "sequence rule" of nomenclature depends on the nature of the groups attached to the asymmetric carbon atom in assigning an (R) or (S) configuration to that carbon atom, it is not possible to state generally that an optically active substituted acyl halide, acyl amine or acid anhydride denoted as (S) will produce an (S) or an (R) product. It can be seen from what has been described previously, that depending on the number of inversions occurring at the asymmetric carbon atom, one may want to begin with an (S) or an (R) substituted acyl halide, acyl amine or acid anhydride in the practice of this invention. However, in view of this disclosure it is considered to be well within the skill of those in the art to which this invention pertains to choose the appropriate optically active starting material to arrive at the desired optically active product.

The present invention is also directed to an optically active stereoisomer of a compound of the formula

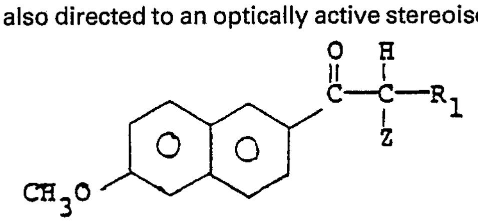

(IV)

wherein $R_1$ is alkyl or cycloalkyl and Z is a leaving group, in the substantial absence of any other stereoisomer of that compound. In the context of this invention, the single stereoisomer of the above-described compounds of formula IV can correspond to 100% optical purity. However, due to the nature of

chemical reactions, a certain amount of another stereoisomer of the compound may be present at the conclusion of the stereoselective preparation due to a small amount of isomerization to the undesired stereoisomer. Accordingly, for the purpose of this invention, the desired optically active stereoisomer is considered to exist in the substantial absence of any other stereoisomers of the compound if the material containing the desired optically active stereoisomer has an optical purity of 90% or more or if such desired stereoisomer is present in greater than 80% enantiomeric excess. The optical purity can be determined by conventional methods by comparing the optical rotation of the material containing the desired stereoisomer to the optical rotation of a purified sample of the desired stereoisomer. The enantiomeric excess can be determined according to conventional methods using nuclear magnetic resonance shift reagents such as tris[3-heptafluoropropylhydroxymethylene)-d-camphorato]europium(III) derivative and tris[3-trifluoromethylhydroxymethylene)-d-camphorato]europium(III) derivative (Aldrich Chemical Co., Milwaukee, Wisconsin, U.S.A.). The enantiomeric excess is the difference between the percent of the desired stereoisomer (enantiomer) and the percent of the undesired stereoisomer(s) (enantiomer) in the material. For example, in a material consisting of 92% of a desired stereoisomer and 8% of an undesired stereoisomer, the enantiomeric excess of the desired stereoisomer would be 84%.

In the majority of instances, only a single center of asymmetry is present in the compounds of formula IV and only two stereoisomers, i.e., the enantiomers, will be present. In those instances, the optically active enantiomer of a compound of formula IV will be present in the substantial absence of the other enantiomer. In another instance, more than one center of asymmetry may exist in the compounds of formula IV. In that instance, the optically active diastereomer will be present in the substantial absence of any other diastereomer of a compound of formula IV.

For example, when Z is methanesulfonyloxy and $R_1$ is methyl, the compound of formula IV will consist of two optically active enantiomers, i.e.,

(R) 1-(6-methoxy-2-naphthyl)-2-methanesulfonyloxypropan-1-one; and

(S) 1-(6-methoxy-2-naphthyl)-2-methanesulfonyloxypropan-1-one.

In this example, an aspect of this invention (useful in the process of Scheme I outlined above) is a material containing

(S) 1-(6-methoxy-2-naphthyl)-2-methanesulfonyloxypropan-1-one in the substantial absence of

(R) 1-(6-methoxy-2-naphthyl)-2-methanesulfonyloxypropan-1-one.

Another aspect of this invention (useful in the process of Scheme II outlined above) is a material containing

(R) 1-(6-methoxy-2-naphthyl)-2-methanesulfonyloxypropan-1-one in the substantial absence of

(S) 1-(6-methoxy-2-naphthyl)-2-methanesulfonyloxypropan-1-one.

However, when Z is d-10-camphorsulfonyloxy and $R_1$ is methyl, the compound of formula IV consists of two diastereomers which are not mirror images of each other (i.e., not enantiomers). In those instances, the composition of the invention consists of one diastereomer in the substantial absence of the other diastereomer.

While the stereoselective process of this invention is primarily utilized to produce an optically active product, which is present as a single stereoisomer in the substantial absence of any other stereoisomer of the product, thus eliminating subsequent resolution steps, the production of a product enriched in or having a major amount of the single stereoisomer as compared to any other stereoisomer of the product is also useful since the economics of any necessary resolution are improved over the case where a racemic mixture is being resolved.

As described previously, the compounds of formula IV are prepared by reacting an optically active substituted acyl halide, acyl amine or acid anhydride with the Grignard of 2-bromo-6-methoxynaphthalene in an ethereal solvent such as tetrahydrofuran, ethyl ether and the like or mixtures thereof. The preparation of the optically active acyl halides, acyl amines and acid anhydrides has been described previously herein.

The present invention is also direct to an optically active stereoisomer of a compound of the formula:

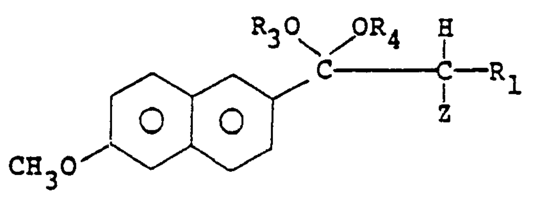

(V)

wherein $R_1$ is alkyl having 1—8 carbon atoms, $R_3$ and $R_4$ are alkyl having 1—8 carbon atoms, optionally the same or different, or when taken together, are alkylene having 2—8 carbon atoms, and Z is halogen, hydroxy, acetoxy, benzoyloxy, dihydropyranyloxy, trialkylsiloxy or a group of the formula:

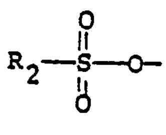

wherein $R_2$ is alkyl, cycloalkyl, alkenyl, alkynyl, aryl or aralkyl, but not d-10-camphoryl when $R_3$ and $R_4$ are

methyl, in the substantial absence of any other stereoisomer of that compound. When only one centre of asymmetry is present in a compound of formula V, the composition consists of a single enantiomer of that compound in the substantial absence of the other enantiomer. When two or more centers of asymmetry are present in a compound of formula V, the composition consists of a single diastereomer in the substantial absence of any other diastereomer of that compound. For the purposes of this invention, the desired optically active stereoisomer is considered to exist in the substantial absence of any other stereoisomers of the compound if the desired optically active stereoisomer has an optical purity of 90% or more.

Any single enantiomer or diastereomer can be utilized in the stereoselective process described herein depending on whether a single or multiple inversion takes place during the sequence of reaction steps to obtain the desired 2-(6-methoxy-2-naphthyl)alkanoic acid.

The invention is further exemplified by the embodiments described in the following illustrative and nonlimiting examples.

### Example 1

120 Grams of (S) ethyl lactate and 120 grams of triethylamine are dissolved in 500 ml. of toluene, with stirring, and the solution is cooled to about 10—15°C. Then, 120 grams of methanesulfonyl chloride are added slowly over a 1½ hour period while maintaining the temperature in the range of 10—15°C. The formation of triethylamine hydrochloride as a precipitate is observed. The solution then is allowed to warm to about 20°C and is poured into water. The aqueous and organic layers are separated and the organic layer is dried over magnesium sulfate and evaporated. The residue remaining is distilled at about 110°C under 2 mm. Hg to afford 161 grams of (S) ethyl 2-methanesulfonyloxypropionate displaying an optical rotation of $[\alpha]^D_{25} = -44°$. That compound displays a characteristic NMR spectra of $\delta$ = 1.28 (triplet, J=2.2), 1.57 (doublet, J=2.3), 3.12, 4.23 (quartet, J=2.2), 5.12 (quartet, J=2.3).

### Example 2

A solution of 75 grams of (S) ethyl 2-methanesulfonyloxypropionate in 250 ml. of methanol and 100 ml. of water is cooled to about 15°C. A 40% aqueous sodium hydroxide solution is slowly added to the above solution to maintain the pH at about 10.5. The reaction proceeds with rapid fall of pH and continued additions of the sodium hydroxide solution are made until the pH falls very slowly or is substantially constant. Then, concentrated hydrochloric acid is added until a pH of 1.9 is obtained. The methanol is removed under reduced pressure and the aqueous layer remaining is extracted with methylene chloride. The organic extract is evaporated to yield, as an oil, 49 grams of (S) 2-methanesulfonyloxypropionic acid, displaying an optical rotation of about $[\alpha]^D_{25} = -53.3°$.

### Example 3

84.85 Grams of (S) ethyl 2-methanesulfonyloxypropionate is dissolved in a solution of 180 ml. of methanol and 80 ml. of water, and the resultant solution is cooled to below −15°C. Then an aqueous 35% sodium hydroxide solution is added slowly to maintain the pH at less than or equal to 10.5 and the addition is continued until the pH remains substantially constant. Concentrated hydrochloric acid is added to acidify the solution to a pH of about 1.8 and the methanol is evaporated under reduced pressure. The aqueous layer is extracted with ethyl acetate several times, and the organic extracts are dried over magnesium sulfate. Evaporation of the organic extract to dryness affords 51 grams of (S) 2-methanesulfonyloxypropionic acid, exhibiting an optical rotation in methylenechloride of $[\alpha]^D_{25} = -54°$. That compound is crystallized from toluene and exhibits a melting point of 72°—75°C and a characteristic NMR spectra of $\delta$ = 1.62 (doublet, J=2.3), 3.09, 5.11 (quartet, J = 2.3), 10.

### Example 4

A mixture containing 40 grams of (S) 2-methanesulfonyloxypropionic acid, 32 grams of thionyl chloride and one drop of dimethylformamide is heated to about 50°C, at which temperature gas evolution is observed. The mixture is slowly heated to about 70°C and maintained at that temperature for about 1 hour. Distillation at 110°C under 1.5 mm. Hg affords 31.2 grams of (S) 2-methanesulfonyloxypropionyl chloride, exhibiting an optical rotation in methylene chloride of $[\alpha]^D_{25} = -36.9°$. That compound exhibits a characteristic NMR spectra of $\delta$ = 1.68 (doublet, J=2.3), 3.15, 5.25 (quartet, J=2.3).

### Example 5

By substituting an equivalent quantity of thionyl bromide in Example 4 and otherwise following the procedure of that Example there is obtained (S) 2-methanesulfonyloxypropionyl bromide.

### Example 6

By substituting an equivalent quantity of p-toluenesulfonyl chloride in Example 1 and following the procedure of that Example with heating at 60°C for 8 hours, and subsequently proceeding according to the manner described in Examples 3 and 4, there is obtained (S) 2-p-toluenesulfonyloxypropionyl chloride. That compound exhibits an $[\alpha]^D_{25} = -32°$ in chloroform and a characteristic NMR spectra of $\delta$ = 1.18 (triplet, J=2.2), 1.48 (doublet, J=2), 2.45, 4.13 (quartet, J=2.2), 4.96 (quartet, J=2), 7.28—8.03 (multiplet).

Example 7

By substituting an equivalent quantity of benzenesulfonyl chloride for the methanesulfonyl chloride of Example 1, and proceeding according to that Example with heating at 30°—40°C for 5—6 hours and subsequently following the procedure of Examples 3 and 4, there is obtained (S) 2-benzenesulfonyloxypropionyl chloride.

Example 8

By substituting an equivalent quantity of (R) ethyl lactate in the procedure of Example 1 and otherwise following that procedure and those described in Examples 2 and 4, there is obtained (R) 2-methanesulfonyloxypropionyl chloride.

Example 9

10 Grams of 2-bromo-6-methoxynaphthalene dissolved in 40 ml. of tetrahydrofuran are slowly added to 3.6 grams of magnesium metal at the refluxing temperature of tetrahydrofuran (about 60—62°C). After the addition is completed, the mixture is stirred at reflux for about 1 hour and the excess magnesium is removed by filtration to afford the Grignard solution [(6-methoxy-2-naphthyl)magnesium bromide in tetrahydrofuran].

Example 10

The solution of (6-methoxy-2-naphthyl)magnesium bromide prepared in Example 9 is slowly added to 8 grams of (S) 2-methanesulfonyloxypropionyl chloride dissolved in 40 ml. of tetrahydrofuran which has been cooled to −40°C, while keeping the temperature of the reaction mixture at about −40°C. The mixture is stirred for an additional hour at that temperature and poured into 200 ml of 5% aqueous hydrochloric acid. 100 Ml. of ethyl ether is added to the reaction mixture. The precipitate is recovered by filtration and washed with 30 ml. of ice cold ethyl ether to yield 6.46 grams of (S) 2-methanesulfonyloxy-1-(6-methoxy-2-naphthyl)propan-1-one, melting at 149—151°C and displaying an optical rotation in chloroform of $[\alpha]^{D}_{25}$ = −33°. That compound displays a characteristic NMR spectra in deutorochloroform of δ = 1.65 (doublet, J=2.1), 3.10, 3.9, 6.17 (quartet, J=2.1), 8.55—7.10 (multiplet).

Example 11

Substitution of an equivalent quantity of (S) 2-p-toluenesulfonyloxypropionyl chloride in the procedure of Example 10 and conducting the coupling at −78°C affords (S) 1-(6-methoxy-2-naphthyl)-2-p-toluenesulfonyloxypropan-1-one. That compound exhibits a melting point of about 117—119°C, an $[\alpha]^{D}_{25}$ = +24.2° in chloroform and a characteristic NMR spectra of δ = 1.67 (doublet, J=2.2), 2.37, 3.98, 5.92 (quartet, J=2.2), 7.14—8.44 (multiplet).

Example 12

Substitution of an equivalent quantity of (S) 2-benzenesulfonyloxypropionyl chloride in the procedure of Example 10 affords (S) 2-benzenesulfonyloxy-1-(6-methoxy-2-naphthyl)propan-1-one.

Example 13

Substitution of an equivalent quantity of (R) 2-methanesulfonyloxypropionyl chloride in the procedure of Example 10 affords (R) 2-methanesulfonyloxy-1-(6-methoxy-2-naphthyl)propan-1-one.

Example 14

Substitution of an equivalent quantity of (R) 2-p-toluenesulfonyloxypropionyl chloride in the procedure of Example 10 affords (R) 1-(6-methoxy-2-naphthyl)-2-p-toluenesulfonyloxypropan-1-one.

Example 15

Substitution of an equivalent quantity of (R) 2-benzenesulfonyloxypropionyl chloride in the procedure of Example 10 affords (R) 2-benzenesulfonyloxy-1-(6-methoxy-2-naphthyl)propan-1-one.

Example 16

A slurry of 4.6 grams of (S) 2-methanesulfonyloxy-1-(6-methoxy-2-naphthyl)propan-1-one in 50 ml. of methanol is treated with 50 grams of trimethyl orthoformate and 2 g of concentrated sulfuric acid. The mixture is heated to about 55°C and maintained at that temperature for about 15 hours. Then the mixture is cooled and poured into aqueous sodium bicarbonate and extracted with 120 ml. of ethyl ether. The organic layer is separated and dried over magnesium sulfate and filtered. Evaporation of the ether under reduced pressure affords 4.8 grams of (S) 1,1-dimethoxy-1-(6-methoxy-2-naphthyl)prop-2-yl methanesulfonate, melting at about 112—115°C and displaying an optical rotation of $[\alpha]^{D}_{25}$ = −23.9° (c=1, chloroform). That compound displays a characteristic NMR spectra in deuterochloroform of τ = 9.0 (doublet, J=2), 6.85, 6.70, 6.61, 6.07, 4.89 (quartet, J=2), 1.99—2.88 (multiplet).

Example 17

To a solution of 7 grams of sodium acetate in 50 ml. of acetic acid is added 3 grams of (S) 1,1-dimethoxy-1-(6-methoxy-2-naphthyl)prop-2-yl methanesulfonate. The mixture is heated to about 110°C for

about 1.5 hours and then poured into 300 ml. of water. The precipitate is recovered by filtration and washed with methanol to afford (S) methyl 2-(6-methoxy-2-naphthyl)propionate, melting at about 85—87°C and displaying an optical rotation of $[\alpha]^{D}_{25} = +65.4°$ (c = 1, chloroform). That material is about 92% optically pure.

Example 18

Substitution of an equivalent quantity of (S) 1-(6-methoxy-2-naphthyl)-2-p-toluenesulfonyloxypropan-1-one in Example 16 and proceeding according to that Example affords (S) 1,1-dimethoxy-1-(6-methoxy-2-naphthyl)prop-2-yl p-toluenesulfonate. Treatment of that material in a manner similar to that described in Example 17 affords (S) methyl 2-(6-methoxy-2-naphthyl)propionate.

Example 19

Substitution of an equivalent quantity of (S) 2-benzenesulfonyloxy-1-(6-methoxy-2-naphthyl)propan-1-one in the procedure of Example 16 and proceeding according to that Example affords (S) 1,1-dimethoxy-1-(6-methoxy-2-naphthyl)prop-2-yl benzenesulfonate. Treatment of that material in a manner similar to that described in Example 17 affords (S) methyl 2-(6-methoxy-2-naphthyl)propionate.

Example 20

The (R) 2-methanesulfonyloxy-1-(6-methoxy-2-naphthyl)-propan-1-one prepared in Example 13 is treated with a 1.5 molar excess of sodium methoxide in methanol solution. That mixture is stirred for about 1 hour at room temperature and the methanol is stripped from the mixture at about 50°C on a rotary evaporator until approximately 80% of the methanol has been removed. The resulting reaction mixture is quenched in water and extracted with methylene chloride. The organic layer is separated, dried over magnesium sulfate and evaporated under reduced pressure to afford (S) 1,1-dimethoxy-1-(6-methoxy-2-naphthyl)propan-2-ol. That material is dissolved in methylene chloride containing triethylamine and the reaction mixture is cooled to about 5°C and one equivalent of methanesulfonyl chloride is added slowly, maintaining the temperature between 5—10°C. After the addition of the methanesulfonyl chloride has been completed, the reaction mixture is stirred for an additional ½ hour. Then the solution is filtered to remove the triethylamine hydrochloride crystals and the filtrate is poured into water. The organic layer is separated and dried over magnesium sulfate. Evaporation of the organic layer under reduced pressure affords (S) 1,1-dimethoxy-1-(6-methoxy-2-napthyl)prop-2-yl methanesulfonate. That material is treated in a manner similar to Example 17 to afford (S) methyl 2-(6-methoxy-2-naphthyl)propionate.

Example 21

An equivalent quantity of each of the following materials: (S) 2-chloropropionyl chloride, (S) 2-bromopropionyl chloride, (R) 2-chloropropionyl chloride and (R) 2-bromopropionyl chloride prepared by the method of Fu et al, *JACS, 76,* 6054 (1954) is substituted for (S) 2-methanesulfonyloxypropionyl chloride in Example 10, and the procedure of that Example is otherwise followed to afford, respectively,

(S) 2-chloro-1-(6-methoxy-2-naphthyl)propan-1-one,
(S) 2-bromo-1-(6-methoxy-2-naphthyl)propan-1-one,
(R) 2-chloro-1-(6-methoxy-2-naphthyl)propan-1-one, and
(R) 2-bromo-1-(6-methoxy-2-naphthyl)propan-1-one.

Example 22

The following compounds are prepared from the compounds of Example 21 in a manner similar to that described in European Patent Office Application No. 81200210.3, filed February 23, 1981 [EPO publication number 0034871, published September 2, 1981]:

(a) From (S) 2-chloro-1-(6-methoxy-2-naphthyl)-propan-1-one:
  (1) (S) 2-chloro-1,1-dimethoxy-1-(6-methoxy-2-naphthyl)propane
  (2) (S) 2-chloro-1,1-diethoxy-(6-methoxy-2-naphthyl)propane
  (3) (S) 2-chloro-1-(6-methoxy-2-naphthyl)-propan-1-one ethylene acetal
  (4) (S) 2-chloro-1-(6-methoxy-2-naphthyl)-propan-1-one propylene acetal
  (5) (S) 2-chloro-1-(6-methoxy-2-naphthyl)-propan-1-one 1,2-dimethylethylene acetal
(b) From (S) 2-bromo-1-(6-methoxy-2-naphthyl)-propan-1-one:
  (1) (S) 2-bromo-1,1-dimethoxy-(6-methoxy-2-naphthyl)propane
  (2) (S) 2-bromo-1,1-diethoxy-(6-methoxy-2-naphthyl)propane
  (3) (S) 2-bromo-1-(6-methoxy-2-naphthyl)propan-1-one ethylene acetal
  (4) (S) 2-bromo-1-(6-methoxy-2-naphthyl)propan-1-one propylene acetal
  (5) (S) 2-bromo-1-(6-methoxy-2-naphthyl)propan-1-one 1,2-dimethylethylene acetal.

Example 23

The materials prepared in Example 22 are each rearranged in a manner similar to that described in EPO Application No. 81200210.3, filed February 23, 1981 [EPO publication no. 0034871, published September 2, 1981], with the following Lewis acids: barium chloride, bismuth chloride, calcium chloride, cadmium chloride, cobalt chloride, cuprous chloride, ferrous chloride, ferric chloride, mercurous chloride,

magnesium chloride, manganese bromide, manganese chloride, nickel bromide, paladium chloride, antimony chloride, stannous chloride, stannic chloride, zinc bromide, zinc chloride and zinc diacetate: to afford, after hydrolysis of any ester formed, (S) 2-(6-methoxy-2-naphthyl)propionic acid.

Example 24

The following compounds prepared in Example 21: (R) 2-chloro-1-(6-methoxy-2-naphthyl)propan-1-one and (R) 2-bromo-1-(6-methoxy-2-naphthyl)propan-1-one are each allowed to react with at least one equimolar amount of sodium methoxide in at least an equimolar amount of methanol, to afford in each instance, (S) 1,1-dimethoxy-1-(6-methoxy-2-naphthyl)propan-2-ol.

Example 25

(S) 1,1-dimethoxy-1-(6-methoxy-2-naphthyl)propan-2-ol is treated with a molar excess (up to 50% excess) of methanesulfonyl chloride in the presence of a molar excess of triethylamine (equal to or greater than the molar excess of methanesulfonyl chloride) in methylene chloride to afford (S) 1,1-dimethoxy-1-(6-methoxy-2-naphthyl)prop-2-yl methanesulfonate. That material is converted to (S) methyl 2-(6-methoxy-2-naphthyl)propionate in a manner similar to that described in Example 17.

Example 26

(S) 1,1-dimethoxy-1-(6-methoxy-2-naphthyl)propan-2-ol is treated with a molar excess of p-toluenesulfonyl chloride in the presence of a molar excess of triethylamine in a manner similar to Example 25 to yield (S) 1,1-dimethoxy-1-(6-methoxy-2-naphthyl)prop-2-yl p-toluenesulfonate. That material is converted to (S) methyl 2-(6-methoxy-2-naphthyl)propionate in a manner similar to that described in Example 17.

Example 27

The process described in Example 26 is repeated by substituting benzenesulfonyl chloride for the p-toluenesulfonyl chloride recited in that Example to afford (S) 1,1-dimethoxy-1-(6-methoxy-2-naphthyl)prop-2-yl benzenesulfonate. That material is converted to (S) methyl 2-(6-methoxy-2-naphthyl)propionate in a manner similar to that described in Example 17.

Example 28

A solution containing 6 ml. of anhydrous methylene chloride and 1.0 millimole of (S) 1,1-dimethoxy-1-(6-methoxy-2-naphthyl)prop-2-yl p-toluenesulfonate is added dropwise to a stirred mixture of 0.20 ml. of iodotrimethylsilane and one drop of cyclohexane in 8 ml. of anhydrous methylene chloride at room temperature under an argon atmosphere. The mixture is stirred for about 1 hour at room temperature and 10 ml. of saturated aqueous sodium bicarbonate is added. The organic and aqueous layers are separated and the organic layer is washed successively with 5 ml. of 10% aqueous sodium thiosulfate, 5 ml. of water, 5 ml. of aqueous sodium bicarbonate and 5 ml. of water. Then the organic layer is dried over magnesium sulfate to afford, upon evaporation of the solvent, (S) methyl 2-(6-methoxy-2-naphthyl)propionate.

Example 29

In a manner similar to that employed in Example 28, (S) 1,1-dimethoxy-1-(6-methoxy-2-naphthyl)prop-2-yl p-toluenesulfonate is converted to (S) methyl 2-(6-methoxy-2-naphthyl)propionate.

Example 30

In a manner similar to that described in Example 28, (S) 1,1-dimethoxy-1-(6-methoxy-2-naphthyl)prop-2-yl benzenesulfonate is converted to (S) methyl 2-(6-methoxy-2-naphthyl)propionate.

Example 31

The materials prepared in Example 22 are each rearranged in a manner similar to that described in British Patent No. 8005752, filed February 20, 1980 [publication number 2042543, published September 24, 1980] with the following silver salts:

(1) silver tetrafluoroborate and $BF_3 \cdot 2CH_3OH$
(2) silver carbonate and $BF_3 \cdot 2CH_3OH$
(3) silver acetate and $BF_3 \cdot 2CH_3OH$
(4) silver oxide and $BF_3 \cdot 2CH_3OH$
(5) silver tetrafluoroborate in methanol.

to yield, after hydrolysis of any ester formed, (S) 2-(6-methoxy-2-naphthyl)propionic acid.

Example 32

A mixture of 90 grams of ethylene glycol, 30 grams of (S) 1-(6-methoxy-2-naphthyl)-2-methanesulfonyloxypropan-1-one, 6 grams of p-toluenesulfonic acid monohydrate and 400 ml. of toluene is heated to reflux. An azeotrope of toluene, water and ethylene glycol is removed, and the water and

ethylene glycol separate upon cooling and are removed via a Dean Stark trap. The reaction mixture is azeotropically dried for 5 hours and then cooled. The cooled mixture is poured into excess aqueous sodium bicarbonate, and the toluene layer is separated and dried over magnesium sulfate.

The toluene is removed by evaporation and the solid remaining is stirred in methanol to yield, after filtration, (S) 1-(6-methoxy-2-naphthyl)-2-methanesulfonyloxy-propan-1-one ethylene acetal, displaying an optical rotation of $[\alpha]^{D}_{25} = +6.1°$ (C=1, chloroform). That compound displays a characteristic NMR spectra in deuterochloroform of δ = 1.35 (doublet, J=2), 2.78, 3.83, 3.98—3.68 (multiplet), 4.28—4.0 (multiplet), 4.98 (quartet, J=2), 8—7 (multiplet).

Example 33

A suitable pressure reactor is charged with 6.4 grams of (S) 1-(6-methoxy-2-naphthyl)-2-methanesulfonyloxypropan-1-one ethylene acetal, 60 ml. of 1,2-diethoxyethane, 50 ml. of water and 3 grams of calcium carbonate. The mixture is heated while being stirred at 120°C for 36 hours at 42 psi. Then the mixture is cooled and the calcium salts removed by filtration. Concentrated hydrochloric acid is added and the mixture is reheated to 95°C for 3 hours. Then the 1,2-diethoxyethane is removed by distillation to yield a solid which is extracted with ethyl ether. The organic layer is back extracted with aqueous sodium bicarbonate and the aqueous and organic layers are separated. The aqueous layer is acidified with hydrochloric acid to afford, after filtration, (S) 2-(6-methoxy-2-naphthyl)propionic acid, exhibiting a melting point of 147°C—150°C and an optical rotation of $[\alpha]^{D}_{25} = +62.2°$ in chloroform.

Example 34

The procedure of Example 33 is repeated using dimethylformamide in place of the 1,2-dimethoxyethane. The mixture is heated to 110°C at atmospheric pressure for 24 hours. After workup, there is obtained (S) 2-hydroxyethyl 2-(6-methoxy-2-naphthyl)propionate by preparative TLC. That material exhibits an $[\alpha]^{D}_{25} = +72.5°$. That material exhibits a characteristic NMR spectra in deuterochloroform of δ = 1.49 (doublet, J=2.3), 3.67 (multiplet), 3.85, 3.89 (quartet, J=2.3), 4.17 (multiplet), 7.77—7.07 (multiplet).

Example 35

A solution of 20.98 mmoles of (S) 2-methanesulfonyloxypropionic acid, 20.95 mmoles of triethylamine and 48 ml. of anhydrous tetrahydrofuran is prepared in a dry vessel under nitrogen and cooled to −30°C. The solution is stirred for about 5 minutes at −30°C, and then 231.23 mmoles of trimethylacetyl chloride are added. A white precipitate is observed. The mixture is allowed to warm to −20°C, and it is stirred at that temperature for 30 minutes. The resulting white slurry is cooled to about −70°C and 20.98 mmoles of the Grignard reagent prepared from 2-bromo-6-methoxy-naphthalene in tetrahydrofuran are added over a one hour period. The mixture is stirred for four hours at −70°C and then allowed to warm to −20°C. Then it is poured into 150 ml. of dilute hydrochloric acid and extracted with methylene chloride. The organic extracts are evaporated to dryness and the remaining material is extracted with ethyl ether. The resulting slurry is filtered, to afford (S) 1-(6-methoxy-2-naphthyl)-2-methanesulfonyloxypropan-1-one, exhibiting a melting point of about 150—154°C.

Example 36

A mixture of 47.2 mmoles of lithium chloride and 21.3 mmoles of manganese chloride in 50 ml. of anhydrous tetrahydrofuran is stirred at 25°C until a yellow solution is formed. Then the Grignard prepared from 19.8 mmoles of 6-methoxy-2-bromonaphthalene in tetrahydrofuran is added at −30°C. That mixture is stirred at −30°C for 1.5 hours and then at 25°C for 20 minutes. The solution of (6-methoxy-2-naphthyl)manganese chloride is added to 22.3 mmoles of (S) 2-methanesulfonyloxypropionyl chloride in 30 ml. of tetrahydrofuran material at −20°C. The mixture is stirred for 1 hour at −20°C, then allowed to warm to 25°C, at which temperature it is stirred for an additional hour. After that time, the mixture is poured into 150 ml. of dilute aqueous hydrochloric acid and extracted with methylene chloride. The methylene chloride is removed by evaporation under reduced pressure and ethyl ether is added. The ethereal slurry is filtered to afford (S) 1 - (6 - methoxy - 2 - naphthyl) - 2 - methanesulfonyloxypropan - 1 - one, melting at about 148—150°C.

Example 37

A dry flask is charged with 80.6 mmoles of imidazole and 50 ml. of anhydrous tetrahydrofuran. Then a solution of 40.3 mmoles of (S) 2-methanesulfonyloxypropionyl chloride in 50 ml. of tetrahydrofuran is added dropwise at room temperature. A white precipitate begins to form during the addition period. The mixture is allowed to stir at room temperature for 2.5 hours, and the resulting white slurry is filtered to remove the imidazole hydrochloride salt. The filtrate, containing 1-(2-methanesulfonyloxypropionyl)imidazole, is cooled to −10°C under nitrogen and 40.0 mmoles of the magnesium Grignard of 2-bromo-6-methoxynaphthalene in tetrahydrofuran is added dropwise at −70°C to −60°C. The mixture is stirred for 40 minutes, allowed to warm to 10°C and poured into 150 ml. of dilute hydrochloric acid. That mixture is extracted with methylene chloride and the organic extracts are evaporated to dryness. The solid is washed with ethyl ether and dried to afford (S) 1 - (6 - methoxy - 2 - naphthyl) - 2 - methanesulfonyloxypropan - 1 - one, exhibiting an $[\alpha]^{D}_{25} = -29.2°$ in methylene chloride.

Example 38

An appropriately sized dry vessel is charged with 20 grams of magnesium shavings and 15 ml. of anhydrous tetrahydrofuran. The stirred mixture is warmed to 50°—60°C and treated with a solution of 16.6 grams of 2-bromo-6-methoxynaphthalene in 35 ml. of anhydrous tetrahydrofuran. Then the mixture is stirred for one hour at 50—60°C. The Grignard solution is transferred to another dry vessel and cooled to 25°. 4.8 Grams of powdered zinc chloride is added to the stirred Grignard solution and the temperature of the mixture is allowed to rise to 45—50°C to afford a solution containing di(6-methoxy-2-naphthyl)zinc.

Example 39

A solution of 15.7 grams of (S) 2-methanesulfonyloxypropionyl chloride in 94 ml. of dry tetrahydrofuran is cooled, with stirring, to −60°C. Then the solution of di(6-methoxy-2-naphthyl)zinc prepared in Example 38 is added over a four hour period. After the addition is completed, the reaction mixture is allowed to warm to 25°C over a 15 hour period. The resulting mixture is added to a stirred mixture containing 30 ml. of concentrated hydrochloric acid and 200 ml. of water. 50 Ml. of diethyl ether is added and the resulting slurry is filtered and dried under reduced pressure at 40°C to yield (S) 1 - (6 - methoxy - 2 - naphthyl) - 2 - methanesulfonyloxypropan - 1 - one.

Example 40

A mixture of 3.07 grams of (S) 1,1 - dimethoxy - 1 - (6 - methoxy - 2 - naphthyl)prop - 2 - yl methanesulfonate, 1.0 gram of calcium carbonate, 100 ml. of dimethylformamide and 25 ml. of water is heated to 110°C and maintained at that temperature for 5 hours. Then the mixture is cooled and the insolubles removed by filtration. The filtrate is poured into excess water and the solid which forms is collected by filtration. Separation by chromatography yields methyl 2-(6-methoxy-2-naphthyl)propionate, exhibiting an $[\alpha]^{D}_{25} = +77°$ in chloroform and an optical purity of greater than 99 percent.

Example 41

A mixture prepared by adding 23 g of d-2-(6-methoxy-2-naphthyl)propionic acid as prepared in Example 23 to 4 g of sodium hydroxide in 500 ml of aqueous methanol was stirred for 3 hours at room temperature. Then the mixture was evaporated to yield sodium 2-(6-methoxy-2-naphthyl)propionate. The product was replaced into toluene then isolated by centrifugation and washed with hexane prior to drying. The product melts at about 255°C with decomposition and its infrared spectrum exhibits maxima at 1260, 1600, 1625 and 1725 $cm^{-1}$. The yield was 95% based on d-2-(6-methoxy-2-naphthyl)propionic acid.

## Claims

1. A process for producing an optically active compound of the formula:

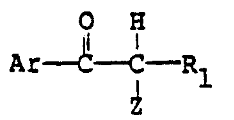

(III)

which comprises contacting an organometallic compound of the formula:

$ArMX$, $(Ar)_2M$ or $ArM'$

with an optically active acyl halide, acyl amine or acid anhydride of the formula:

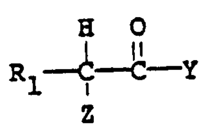

(II)

wherein Ar is aryl, M is cadmium, copper(II), manganese(II), magnesium or zinc, M′ is copper(I) or lithium, $R_1$ is alkyl or cycloalkyl, X is halogen, Y is halogen, the group of the formula:

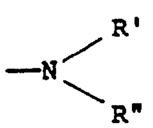

wherein R′ and R″ are alkyl or aryl or when taken together with N form a heterocyclic moiety which optionally can contain other hetero atoms, or acyloxy and Z is a leaving group or a group convertible to a leaving group.

2. A process for the preparation of an optically active α-aryl alkanoic acid of the formula

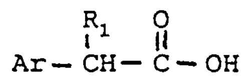

wherein Ar is aryl and $R_1$ is alkyl or cycloalkyl; or a pharmaceutically acceptable salt, ester, ortho ester or amide thereof; in which a compound of formula III is first produced according to the process of claim 1, and then

(A) the compound of formula III is ketalized to form the corresponding optically active aryl alkyl ketal; and the ketal is rearranged to form a said α-aryl alkanoic acid, or an ester, ortho ester or amide thereof; or

(B) the compound of formula III is contacted with a ketalizing agent to form a first optically active ketal of the formula

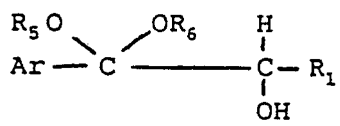

wherein $R_5$ and $R_6$ are alkyl, aryl or aralkyl, optionally the same or different, or when taken together are alkylene having 2 to 8 carbon atoms; a leaving group is regenerated at the α-position of the first ketal to form a second optically active ketal of the formula

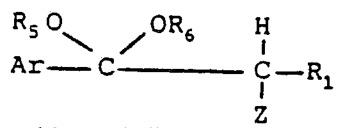

the second ketal is arranged to form a said α-aryl alkanoic acid, or an ester, ortho ester or amide thereof; or

(C) the optically active compound of formula III is heated with a ketalizing agent in the presence of an acid at an elevated temperature for a time sufficient to afford a said α-aryl alkanoic acid; and optionally

(D) any said ester, ortho ester or amide of route (A) or route (B) is hydrolyzed to the corresponding acid, or

(E) said acid of steps (A) to (D) is converted to a pharmaceutically acceptable salt thereof.

3. A process according to claim 1 or claim 2 wherein said organometallic compound is ArMX or $(Ar)_2M$ where M is manganese(II) or zinc.

4. A process according to claim 1 or claim 2 wherein said organometallic compound is ArMX where M is magnesium.

5. A process for producing an optically active α-aryl alkanoic acid of formula

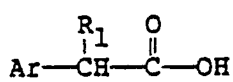

or a pharmaceutically acceptable salt, ester, ortho ester or amide thereof, wherein $R_1$ is alkyl or cycloalkyl and Ar is aryl, from an optically active compound of the formula

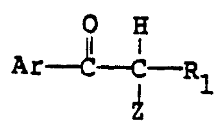 (III)

wherein Z is a leaving group or a group convertible into a leaving group; which comprises:

(A) ketalizing a compound of formula III to form the corresponding optically active aryl alkyl ketal; and rearranging the ketal to form a said α-aryl alkanoic acid, or an ester, ortho ester or amide thereof; or

(B) contacting a compound of formula III with a ketalizing agent to form a first optically active ketal of the formula

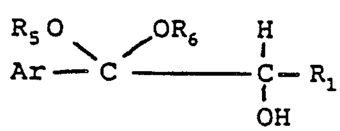

wherein $R_5$ and $R_6$ are alkyl, aryl or aralkyl, optionally the same or different, or when taken together are alkylene having 2 to 8 carbon atoms; regenerating a leaving group at the α-position of the first ketal to form a second optically active ketal of the formula

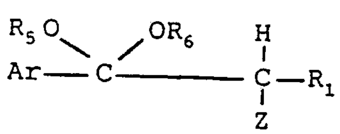

and rearranging the second ketal to form a said α-aryl alkanoic acid, or an ester, ortho ester or amide thereof with the proviso that when $R_5$ and $R_6$ are alkyl Z in the second ketal is other than sulfonyloxy; or

(C) heating an optically active compound of formula III with a ketalizing agent in the presence of an acid at an elevated temperature for a time sufficient to afford a said α-aryl alkanoic acid; and optionally

(D) hydrolyzing any said ester, ortho ester or amide of route (A) or route (B) to the corresponding acid, and further optionally

(E) converting said α-aryl alkanoic acid of steps (A) to (D) to a pharmaceutically acceptable salt thereof.

6. A process for producing an optically active ketal of an optically active compound of the formula

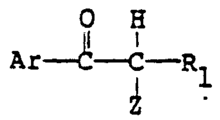

(III)

wherein Ar is aryl, $R_1$ is alkyl or cycloalkyl, and Z is a leaving group or a group convertible to a leaving group with the proviso that when Z is halogen or sulfonyloxy, then the ketal is other than a dialkyl ketal in which Z is hydroxy or sulfonyloxy; which comprises contacting the compound of formula III of which at least a major amount has a preselected absolute configuration with a ketalizing agent effective to maintain said preselected absolute configuration.

7. A process according to claim 2(A), claim 5(A) or claim 6 wherein the ketalizing agent is an ortho ester or a polyhydric alcohol.

8. A process according to claim 7 wherein the ortho ester is a trialkyl orthoformate.

9. A process according to claim 7 wherein the ketalizing agent is trimethyl orthoformate, triethyl orthoformate, ethylene glycol, trimethylene glycol or 2,2-dimethyl-1,3-propanediol.

10. A process according to claim 2(A), claim 2(B), claim 5(A) or claim 5(B) wherein said rearrangement is conducted by solvolysis or in the presence of an agent having affinity for oxygen or in the presence of a silver(I) salt of an organic or inorganic anion in an acidic alcoholic medium.

11. A process according to claim 10 wherein the agent having affinity for oxygen is selected from iodotrialkylsilanes, trialkyl perfluoroalkylsulfonates and Lewis acids.

12. A process according to claim 11 wherein the Lewis acid is selected from acetate, propionate, benzoate, trifluoromethanesulfonate, methanesulfonate, chloride, iodide, bromide and sulfate salts of copper, magnesium, calcium, zinc, cadmium, barium, mercury, tin, antimony, bismuth, manganese(II), iron, cobalt, nickel and palladium.

13. A process according to claim 10 wherein said agent having affinity for oxygen is iodotrimethylsilane, trimethylsilyl, trifluoromethanesulfonate, trimethylsilyl pentafluoromethanesulfonate, aluminum chloride, aluminum bromide, zinc chloride, stannous chloride, stannic chloride, titanium chloride, boron fluoride, ferric chloride or ferrous chloride.

14. A process according to claim 10 wherein the silver(I) salt is selected from silver tetrafluoroborate, silver carbonate, silver acetate and silver oxide.

15. A process according to claim 10 wherein the solvolysis is conducted in the presence of a protic or dipolar aprotic solvent or in the presence of a salt of an organic or inorganic anion.

16. A process according to claim 15 wherein said salt is selected from calcium, sodium, potassium, and lithium salts of carbonate, anions of organic acids and phosphates.

17. A process according to claim 2(B) or claim 5(B) wherein said compound of formula III is ketalized by means of an alkali metal alkoxide, aryloxide or aralkyloxide.

18. A process according to claim 2(C) or claim 5(C) wherein said acid is sulfuric acid.

19. A process according to claim 18 wherein said ketalizing agent is an ortho ester.

20. A process according to claim 19 wherein said ortho ester is a trialkyl orthoformate.

21. A process according to claim 20 wherein said trialkyl orthoformate is trimethyl orthoformate or triethyl orthoformate.

22. A process according to any one of claims 18 to 21 wherein said elevated temperature is at least 80°C.

23. A process according to any one of the preceding claims wherein Ar is 6-methoxy-2-naphthyl and $R_1$ is methyl.

24. A process according to any one of the preceding claims wherein Z is selected from hydroxy, acetoxy, benzoyloxy, dihydropyranyloxy, trialkylsiloxy, halogen and a group of the formula

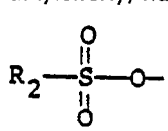

wherein $R_2$ is alkyl, cycloalkyl, alkenyl, alkynyl, aryl or arylalkyl.

25. A process according to claim 24 wherein Z is selected from alkylsulfonyloxy, in particular methanesulfonyloxy, arylsulfonyloxy, in particular benzenesulfonyloxy and p-toluenesulfonyloxy, and aralkylsulfonyloxy.

26. A process according to any one of claims 1 to 5 and 7 to 25 (except as appendent to claim 6) wherein said acyl halide, acyl amine or acid anhydride (in the case of the process of claim 1 or claim 2) or the compound of formula III (in the case of the process of claim 5) is a single stereoisomer in the substantial absence of any other stereoisomer of the compound, or a single stereoisomer of preselected absolute configuration, and the reactions are carried out under conditions which avoid substantial racemization, whereby the desired end product compound is optically active, a single stereoisomer in the substantial absence of any other stereoisomer of that compound, or a single stereoisomer of preselected absolute configuration.

27. A process according to claim 26, wherein Ar is 6-methoxy-2-naphthyl and $R_1$ is methyl, and at least the desired end product has the (S) configuration.

28. A material comprising a single stereoisomer of a cyclic ketal of a compound of the formula

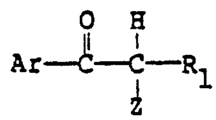

(III)

in the substantial absence of any other stereoisomer of said compound, wherein Ar is substituted or unsubstituted 6-methoxy-2-naphthyl, $R_1$ is alkyl or cycloalkyl, and Z is a leaving group or a group convertible to a leaving group selected from halogen, acetoxy, benzoyloxy, dihydropyranyloxy or trialkylsiloxy.

29. A material according to claim 28 wherein Z is halogen.

30. A material according to claim 29 wherein Z bromine.

31. A material according to any one of claims 28 to 30 wherein Ar is 5-bromo-6-methoxy-2-naphthyl.

32. An optically active cyclic ketal having an absolute (S)-configuration of the formula:

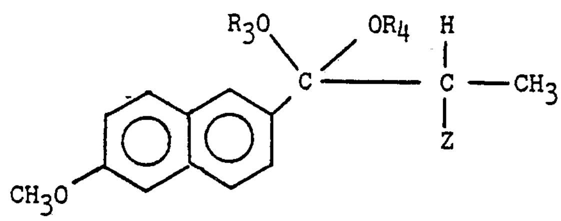

wherein $R_3$ and $R_4$ together provide an alkylene having 2—8 carbon atoms, and Z is halogen, acetoxy, benzoyloxy, dihydropyranyloxy or trialkylsiloxy.

33. A process for the preparation of an optically active α-aryl alkanoic acid of the formula

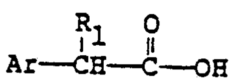

wherein Ar and $R_1$ are as defined in claim 28, or a pharmaceutically acceptable salt, ester, ortho ester or amide thereof; which process comprises the rearrangement of an optically active cyclic ketal of any one of claims 28 to 32.

34. A process according to claim 33 wherein Ar is 6-methoxy-2-naphthyl and $R_1$ is methyl.

35. A process according to claim 34 wherein (S) 2-(6-methoxy-2-naphthyl) propionic acid or a pharmaceutically acceptable salt, ester, ortho ester or amide thereof is prepared.

36. A process according to claim 35 wherein (S) 2-(6-methoxy-2-naphthyl) propionic acid or its sodium salt is prepared.

37. A material comprising a single stereoisomer of a compound of the formula

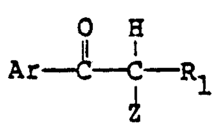

(III)

in the substantial absence of any other stereoisomer of said compound, wherein Ar is 6-methoxy-2-naphthyl, $R_1$ is alkyl or cycloalkyl and Z is a leaving group or a group convertible to a leaving group selected from hydroxy, acetoxy, benzoyloxy, dihydropyranyloxy, trialkylsiloxy or a group of the formula:

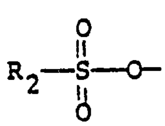

wherein $R_2$ is cycloalkyl, alkenyl or alkynyl.

38. A material comprising a single stereoisomer of a compound of the formula

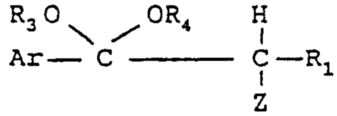

in the substantial absence of any other stereoisomer of said compound, wherein Ar is 6-methoxy-2-naphthyl, $R_1$ is alkyl or cycloalkyl, $R_3$ and $R_4$ are alkyl, optionally the same or different, or when taken together, are alkylene having 2—8 carbon atoms, and Z is a leaving group or a group convertible to a leaving group, in particular a group selected from the class of halogen, acetoxy, benzoyloxy, dihydropyranyloxy or trialkylsiloxy.

39. A material according to claim 38 wherein $R_3$ and $R_4$ are both methyl or ethyl, or together are the ethylene, trimethylene or 2,2-dimethyltrimethylene radical.

40. A material according to any one of claims 37 to 39 wherein Z is selected from hydroxy, chloro, bromo, trimethylsiloxy or triethylsiloxy.

41. A material of claim 38 wherein said single stereoisomer is selected from
(R) or (S) 2-chloro-1,1-dimethoxy-1-(6-methoxy-2-naphthyl)propane,
(R) or (S) 2-bromo-1,1-dimethoxy-1-(6-methoxy-2-naphthyl)propane,
(R) or (S) 2-chloro-1,1-diethoxy-1-(6-methoxy-2-naphthyl)propane,
(R) or (S) 2-bromo-1,1-diethoxy-1-(6-methoxy-2-naphthyl)propane,
(R) or (S) 2-chloro-(6-methoxy-2-naphthyl)propan-1-one ethylene acetal,
(R) or (S) 2-bromo-1-(6-methoxy-2-naphthyl)propan-1-one ethylene acetal.

42. A material according to any one of claims 37 to 41 wherein said stereoisomer has the (S) configuration.

43. A material according to any one of claims 39, 40, and 42 wherein Ar is 6-methoxy-2-napthyl and $R_1$ is methyl.

**Patentansprüche**

1. Verfahren zur Herstellung einer optisch aktiven Verbindung der Formel

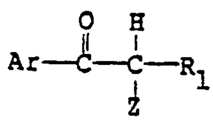

(III)

welches umfaßt das Kontaktieren einer organometallischen Verbindung der Formel:

ArMX, $(Ar)_2M$ oder ArṀ'

mit einem optisch aktiven Acylhalogenid, Acylamin oder Säureanhydrid der Formel

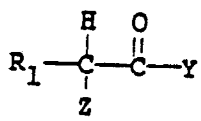

(II)

worin Ar Aryl ist, M Cadmium, Kupfer (II), Mangan (II), Magensium oder Zink ist, M' Kupfer (I) oder Lithium ist, $R_1$ Alkyl oder Cycloalkyl ist, X Halogen ist, Y Halogen, die Gruppe der Formel:

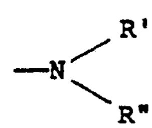

worin R' und R'' oder Aryl sind oder zusammen mit N eine heterocyclishe Gruppe bilden, die gegebenenfalls andere Heteroatome enthalten kann, oder Acyloxy bedeutet und Z eine abspaltbare Gruppe oder eine in eine abspaltbare Gruppe überführbare Gruppe ist.

2. Verfahren zur Herstellung einer optisch aktiven α-Arylalkansäure der Formel

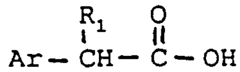

worin Ar Aryl ist und $R_1$ Alkyl oder Cycloalkyl ist; oder eines pharmazeutisch verträglichen Salzes, Esters, Orthoesters oder Amids davon; bei dem man eine Verbindung der Formel III zunächst nach dem Verfahren von Anspruch 1 herstellet und dann

(A) die Verbindung der Formel III zu dem entsprechenden optisch aktiven Arylalkyketal ketalisiert; und das Ketal zu der α-Arylalkansäure oder einem Ester, Orthoester oder Amid davon umlagert; oder

(B) die Verbindung der Formel III mit einem Ketalisierungsmittel kontaktiert, um ein erstes optisch aktives Ketal der Formel

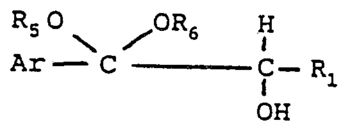

zu bilden, worin $R_5$ und $R_6$ Alkyl, Aryl oder Aralkyl sind, die wahlweise gleich oder verschieden sein können, oder zusammengenommen Alkylen mit 2 bis 8 Kohlenstoffatomen sind; eine abspaltbare Gruppe in der α-Stellung des ersten ketals regeneriert, um ein zweites optisch aktives der Ketal Formel

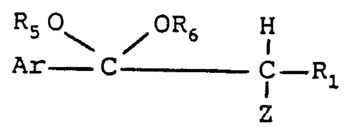

zu bilden, das zweite Ketal zu der α-Arylalkansäure oder einem Ester, Orthoester oder Amid davon umlagert; oder

(C) die optisch aktive Verbindung der Formel III mit einen Ketalisierungsmittel in Gegenwart einer Säure bei erhöhter Temperatur ausreichend lange erwärmt, um die α-Arylalkansäure zu ergeben; und gegebenenfalls

(D) irgendeinen der Ester, Orthoester oder Amide von Weg (A) oder Weg (B) zu der entsprechenden Säure hydrolysiert oder

(E) die Säure der Stufen (A) bis (D) ein pharmazeutisch verträgliches Salz davon überführt.

3. Verfahren nach Anspruch 1 oder 2, worin die organometallische Verbindung ArMX oder $(Ar)_2M$ ist, wobei M Mangan (II) oder Zink ist.

4. Verfahren nach Anspruch 1 oder 2, worin die organometallische Verbindung ArMX ist, wobei M Magnesium ist.

5. Verfahren zu Herstellung einer optisch aktiven α-Arylalkansäure der Formel

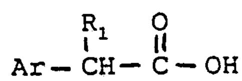

oder eines pharmazeutisch verträglichen Salzes, Esters, Orthoeasters oder Amids davon, worin $R_1$ Alkyl oder Cycloalkyl ist und Ar Aryl ist, aus einer optisch aktiven Verbindung der Formel

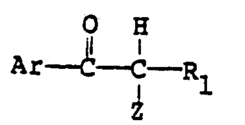

(III)

worin Z eine abspaltbare Gruppe oder eine in eine abspaltbare Gruppe überführbare Gruppe ist; welches umfaßt;

(A) Ketalisieren einer Verbindung der Formel III zu dem entsprechenden optisch aktiven Arylalkylketal; und Umlagern des Ketals zu der α-Arylalkansäure oder einem Ester, Orthoester oder Amid davon; oder

(B) Kontaktieren einer Verbindung der Formel III mit einem Ketalisierungsmittel unter Bildung eines ersten optische aktiven Ketals der Formel

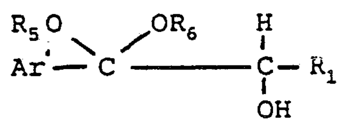

worin $R_5$ und $R_6$ Alkyl, Aryl oder Aralkyl sind, die wahlweise gleich oder verschieden sind, oder zusammengenommen Alkylen mit 2 bis 8 Kohlenstoffatomen sind; Regenerieren einer abspaltbaren Gruppe in der α-Stellung des ersten Ketals unter Bildung eines zweiten optisch aktiven Ketals der Formel

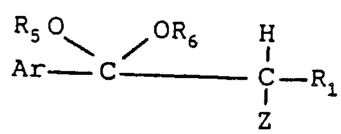

und Umlagern des zweiten Ketals zu der α-Arylalkansäure oder einem Ester, Orthoester oder Amid davon, mit der Maßgabe, daß wenn $R_5$ und $R_6$ Alkyl sind, Z in dem zweiten Ketal von Sulfonyloxy verschieden ist; oder

(C) Erwärmen einer optisch aktiven Verbindung der Formel III mit einem Ketalisierungsmittel in Gegenwart einer Säure bei erhöhter Temperatur und ausreichend lange, um die α-Arylalkansäure zu ergeben; und gegebenenfalls

(D) Hydrolysieren irgendeines der Ester, Orthoester oder Amide von Weg (A) oder Weg (B) zu der entsprechenden Säure und weiter gegebenenfalls

(E) Überführen der α-Arylalkansäure der Stufen (A) bis (D) in ein pharmazeutisch verträgliches Salz davon.

6. Verfahren zur Herstellung eines optisch aktiven Ketals einer optisch aktiven Verbindung der Formel

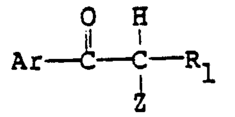

(III)

worin Ar Aryl ist, $R_1$ Alkyl oder Cycloalkyl ist und Z eine abspaltbare Gruppe oder eine in eine abspaltbare Gruppe überführbare Gruppe ist, mit der Maßgabe, daß wenn Z Halogen oder Sulfonyloxy ist, das Ketal kein Dialkylketal ist, bei dem Z Hydroxy oder Sulfonyloxy ist; welches umfaßt das Kontaktieren der Verbindung der Formel III, von der zumindest eine überwiegende Menge eine vorbestimmte absolute Konfiguration hat, mit einem Ketalisierungsmittel, das zur Bewahrung der vorbestimmten absoluten Konfiguration fähig ist.

7. Verfahren nach Anspruch 2(A), Anspruch 5(A) oder Anspruch 6, worin das Ketalisierungsmittel ein Orthoester oder ein mehrwertier Alkohol ist.

8. Verfahren nach Anspruch 7, worin der Orthoester ein Trialkylorthoformiat ist.

9. Verfahren nach Anspruch 7, worin das Ketalisierungsmittel Trimethylorthoformiat, Triethylorthoformiat, Ethylenglykol, Trimethylenglykol oder 2,2-Dimethyl-1,3-propandiol ist.

10. Verfahren nach Anspruch 2(A), Anspruch 2(B), Anspruch 5(A) oder Anspruch 5(B), worin die Umlagerung durch Solvolyse oder in Gegenwart eines Mittels mit Affinität für Sauerstoff oder in Gegenwart eines Silber(I)-Salzes eines organischen oder anorganischen Anions in einem sauren, alkoholischen Medium durchgeführt wird.

11. Verfahren nach Anspruch 10, worin das Mittel mit Affinität für Sauerstoff ausgewählt ist unter Jodtrialkylsilanen, Trialkylperfluoralkylsulfonaten und Lewis-Säuren.

12. Verfahren nach Anspruch 11, worin die Lewis-Säure ausgewählt ist under Acetat, Propionat-, Benzoat-, Trifluormethansulfonat-, Methansulfonat-, Chlorid-, Jodid-, Bromid- und Sulfatsalzen von Kupfer, Magnesium, Calcium, Zink, Cadmium, Barium, Quecksilber, Zinn, Antimon, Wismut, Mangan(II), Eisen, Kobalt, Nickel und Palladium.

13. Verfahren nach Anspruch 10, worin das Mittel mit Affinität für Sauerstoff Jodtrimethylsilan, Trimethylsilyltrifluormethanesulfonat, Trimethylsilylpentafluormethansulfonat, Aluminiumchlorid, Aluminiumbromid, Zinkchlorid, Zinn(II)-chlorid, Zinn(IV)-chlorid, Titanchlorid, Borfluorid, Eisen(III)-chlorid oder Eisen(II)-chlorid ist.

14. Verfahren nach Anspruch 10, worin das Silber(I)-Salz ausgewählt ist unter Silbertetrafluoroborat, Silbercarbonat, Silberacetat und Silberoxid.

15. Verfahren nach Anspruch 10, worin die Solvolyse in Gegenwart eines protischen oder dipolaren aprotischen Lösungsmittels oder in Gegenwart eines Salzes eines organischen oder anorganischen Anions durchgeführt wird.

16. Verfahren nach Anspruch 15, worin das Salz ausgewählt ist under Calcium-, Natrium-, Kalium- und Lithiumsalzen von Carbonat, Bicarbonat, Anionen von organischen Säuren und Phosphaten.

17. Verfahren nach Anspruch 2(B) oder Anspruch 5(B), worin die Verbindung der Formel III mit Hilfe eines Alkalimetallalkoxids, -aryloxids oder -aralkyloxids ketalisiert wird.

18. Verfahren nach Anspruch 2(C) oder Anspruch 5(C), worin die Säure Schwefelsäure ist.

19. Verfahren nach Anspruch 18, worin das Ketalisierungsmittel ein Orthoester ist.

20. Verfahren nach Anspruch 19, worin der Orthoester ein Trialkylorthoformiat ist.

21. Verfahren nach Anspruch 20, worin das Trialkylorthoformiat Trimethylorthoformiat oder Triethylorthoformiat ist.

22. Verfahren nach irgendeinem der Anprüche 18 bis 21, worin die erhöhte Temperatur mindestens 80°C beträgt.

23. Verfahren nach irgendeinem der vorangehenden Ansprüche, worin Ar 6-Methoxy-2-naphthyl ist und $R_1$ Methyl ist.

24. Verfahren nach irgendeinem der vorangehenden Ansprüche, worin Z ausgewählt ist unter Hydroxy, Acetoxy, Benzolyoxy, Dihydropyranyloxy, Trialkylsiloxy, Halogen und einer Gruppe der Formel

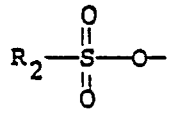

worin $R_2$ Alkyl, Cycloalkyl, Alkenyl, Alkinyl, Aryl oder Arylalkyl ist.

25. Verfahren nach Anspruch 24, worin Z ausgewählt ist under Alkylsulfonyloxy, insbesondere Methansulfonyloxy, Arylsulfonyloxy, insbesondere Benzolsulfonyloxy und p-Toluolsulfonyloxy, und Aralkysulfonyloxy.

26. Verfahren nach irgendeinem der Ansprüche 1 bis 5 und 7 bis 25, (ausgenommen, soweit sie von Anspruch 6 abhängig sind), worin das Acylhalogenid, Acylamin oder Säureanhydrid (im Falle des Verfahrens von Anspruch 1 oder Anspruch 2) oder die Verbindung der Formel III (im Falle des Verfahrens von Anspruch 5) ein einziges Stereoisomer in praktischer Abwesenheit irgendeines anderen Stereoisomers der Verbindung ist oder ein einziges Stereoisomer von vorbestimmter absoluter Konfiguration ist, und die Reaktionen unter Bedingungen durchgeführt werden, die eine nennenswerte Racemisierung vermeiden, wodurch das gewünschte Endprodukt optisch aktiv, ein einziges Stereoisomer in praktischer Abwesenheit irgendeines anderen Stereoisomers dieser Verbindung oder ein einziges Stereoisomer von vorbestimmter absoluter Konfiguration ist.

27. Verfahren nach Anspruch 26, worin Ar 6-Methoxy-2-naphthyl ist und $R_1$ Methyl is und zumindest das gewünschte Endprodukt die (S)-Konfiguration hat.

28. Material, umfassend ein einziges Stereoisomer eines cyclischen Ketals einer Verbindung der Formel

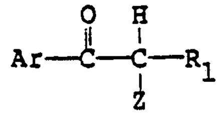

(III)

in praktischer Abwesenheit irgendeines anderen Stereoisomers der Verbindung, worin Ar substituiertes oder unsubstituiertes 6-Methoxy-2-naphthyl ist, $R_1$ Alkyl oder Cycloalkyl is und Z eine abspaltbare Gruppe oder eine in eine abspaltbare Gruppe überführbare Gruppe ist, die ausgewählt ist unter Halogen, Acetoxy, Benzoyloxy, Dihydropyranyloxy oder Trialkylsiloxy.

29. Material nach Anspruch 28, worin Z Halogen ist.

30. Material nach Anspruch 29, worin Z Brom ist.

31. Material nach irgendeinem der Ansprüche 28 bis 30, worin Ar 5-Brom-6-methoxy-2-naphthyl ist.

32. Optisch aktives cyclisches Ketal mit absoluter (S)-Konfiguration der Formel:

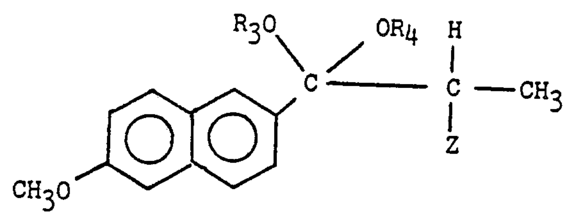

worin $R_3$ und $R_4$ zusammen ein Alkylen mit 2 bis 8, Kohlenstoffatomen ergeben und Z Halogen, Acetoxy, Benzoyloxy, Dihydropyranyloxy oder Trialkylsiloxy ist.

33. Verfahren zur Herstellung einer optisch aktiven α-Arylalkansäure der Formel

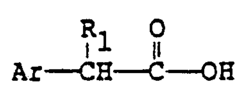

worin Ar und $R_1$ wie in Anspruch 28 definiert sind, oder eines pharmazeutisch verträglichen Salzes, Esters, Orthoesters oder Amids davon; welches Verfahren umfaßt die Umlagerung eines optisch aktiven, cyclischen Ketals nach irgendeinem der Ansprüche 28 bis 32.

34. Verfahren nach Anspruch 33, worin Ar 6-Methoxy-2-naphthyl ist und $R_1$ Methyl ist.

35. Verfahren nach Anspruch 34, worin (S)-2-(6-Methoxy-2-naphthyl)-propionsäure oder ein pharmazeutisch verträgliches Salz, Ester, Orthoester oder Amid davon hergestellt wird.

36. Verfahren nach Anspruch 35, worin (S)-2-(6-Methoxy-2-naphthyl)-propionsäure oder dessen Natriumsalz hergestellt wird.

37. Material, umfassend ein einziges Stereoisomer einer Verbindung der Formel

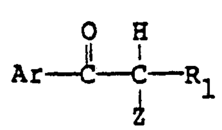

(III)

in praktischer Abwesenheit irgedeines anderen Stereoisomers der verbindung, worin Ar 6-Methoxy-2-naphthyl ist, $R_1$ Alkyl oder Cycloalkyl ist und Z eine abspaltbare Gruppe oder eine in eine abspaltbare

Gruppe überfuhrbare Gruppe ist, die ausgewählt ist unter Hydroxy, Acetoxy, Benzoyloxy, Dihydropyranyloxy, Trialkylsiloxy oder einer Gruppe der Formel

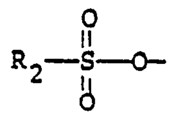

worin $R_2$ Cycloakyl, Alkenyl oder Alkinyl ist.

38. Material, umfassend ein einziges Stereoisomer einer Verbindung der Formel

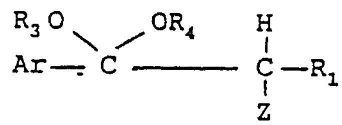

in praktischer Abwesenheit irgendeines anderen Stereoisomers der Verbindung, worin Ar 6-Methoxy-2-naphthyl ist, $R_1$ Alkyl oder Cycloalkyl ist, $R_3$ und $R_4$ Alkyl sind, die wahlweise gleich oder verschieden sind, oder zusammen Alkylen mit 2 bis 8 Kohlenstoffatomen sind und Z eine abspaltbare Gruppe oder eine in eine abspaltbare Gruppe überführbare Gruppe ist, insbesondere eine unter Halogen, Acetoxy, Benzoyloxy Dihydropyranyloxy oder Trialkylsiloxy ausgewählte Gruppe.

39. Material nach Anspruch 38, worin $R_3$ und $R_4$ beide Methyl oder Ethyl sind oder zusammen Ethylen, Trimethylen oder 2,2-Dimethyltrimethylen sind.

40. Material nach irgendeinem der Ansprüche 37 bis 39, worin Z ausgewählt ist unter Hydroxy, Chlor, Brom, Trimethylsiloxy oder Triethylsiloxy.

41. Material nach Anspruch 38, worin das einzige Stereoisomer ausgewählt ist unter:
(R)- oder (S)-2-Chlor-1,1-dimethoxy-1-(6-methoxy-2-naphthyl)-propan,
(R)- oder (S)-2-Brom-1,1-dimethoxy-1-(6-methoxy-2-naphthyl)-propan,
(R)- oder (S)-2-Chlor-1,1-diethoxy-1-(6-methoxy-2-naphthyl)-propan,
(R)- oder (S)-2-Brom-1,1-diethoxy-1-(6-methoxy-2-napthyl)-propan,
(R)- oder (S)-2-Chlor-1-(6-methoxy-2-naphthyl)-propan-1-on-ethylenacetal,
(R)- oder (S)-2-Brom-1-(6-methoxy-2-naphthyl)-propan-1-on-ethylenacetal.

42. Material nach irgendeinem der Ansprüche 37 bis 41, worin das Stereoisomer die (S)-Konfiguration hat.

43. Material nach irgendeinem der Ansprüche 39, 40 und 42, worin Ar 6-methoxy-2-naphthyl ist und $R_1$ Methyl ist.

## Revendications

1. Procédé de production d'un composé optiquement actif der formule:

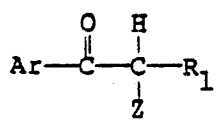

(III)

qui consiste à faire entrer und composé organométallique de formule:

ArMx, $(Ar)_2M$ ou ArM′

en contact avec un halogénure d'acyle, une acylamine ou un anhydride d'acide optiquement actifs de formule:

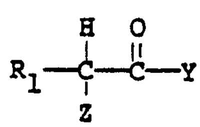

(II)

dans laquelle Ar est un groupe aryle, M est le cadmium, le cuivre(II), le manganèse(II), le magnésium ou le zinc, M′ est le cuivre(I) ou le lithium, $R_1$ est un groupe alyle ou cycloalkyle, X est un halogène, Y est un halogène, le groupe de formule:

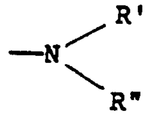

dans laquelle R′ et R″ sont des radicaux alkyle ou aryle ou bien R′ et R″ forment conjointement avec l'atome d'azote un groupement hétérocyclique qui peut facultativement contenir d'autres hétéro-atomes, ou un groupe acyloxy, et Z est un groupe partant ou un groupe pouvant être transformé en un groupe partant.

2. Procédé de préparation d'un acide α-arylalcanoïque optiquement actif de formule

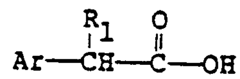

dans laquelle Ar est un groupe aryle et $R_1$ est un groupe alkyle ou cycloalkyle, ou d'un sel, ester, orthoester ou amide pharmaceutiquement acceptable de cet acide; dans lequel un composé de formule III est tout d'abord produit conformément au procédé suivant la revendication 1, puis

(A) le composé de formule III est cétalisé pour former l'aryalkylcétal optiquement actif correspondant; et le cétal est transposé pour former un acide α-arylalcanoïque ou un ester, ortho-ester ou amide de cet acide; ou bien

(B) le composé de formule III est mis en contact avec un agent de cétalisation pour former un premier cétal optiquement actif de formule

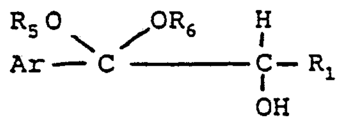

dans laquelle $R_5$ et $R_6$ sont des groupes alkyle, aryle ou aralkyle facultativement identiques ou différents ou forment conjointement un groupe alkylène ayant 2 à 8 atomes de carbone; un groupe partant est régénéré dans la position α du premier cétal pour former second cétal optiquement actif de formule

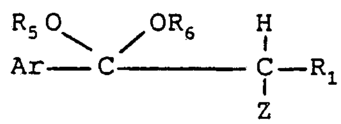

le second cétal est transposé pour former un acide α-arylalcanoïque ou un ester, un ortho-ester ou un amide de cet acide; ou bien

(C) le composé optiquement actif de formule III est chauffé avec un agent de cétalisation en présence d'un acide à une température élevée pendant une durée suffisante pour obtenir un acide α-arylalcanoïque tel que défini; et à titre facultatif

(D) à hydrolyser tout ester, ortho-ester ou amide de la voie d'obtention (A) ou (B) en l'acide correspondant, ou bien

(E) à convertir ledit acide des étapes (A) à (D) en un sel pharmaceutiquement acceptable.

3. Procédé suivant la revendication 1 ou 2, dans lequel ledit composé organométallique répond à la formule ArMX ou $(Ar)_2M$ dans laquelle M est le manganèse(II) ou le zinc.

4. Procédé suivant la revendication 1 ou la revendication 2, dans lequel le composé organométallique répond à la formule ArMX où M désigne le magnésium.

5. Procédé de production d'un acide α-arylalconoïque optiquement actif de formule

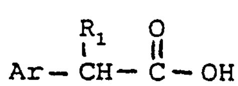

ou d'un sel, ester, ortho-ester, ou amide pharmaceutiquement acceptable de cet acide, formule dans laquelle $R_1$ est un groupe alkyle ou cycloalkyle et Ar est un groupe aryle, à partir d'un composé optiquement actif de formule

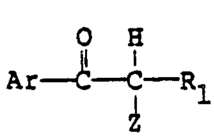

(III)

dans laquelle Z est un groupe partant ou un groupe pouvant être transformé en un groupe partant; procédé qui consiste:

(A) à cétaliser un composé de formule III pour former l'arylalkylcétal optiquement actif correspondant; et à transposer le cétal pour former un acide α-arylalcanoïque ou un ester, ortho-ester ou amide cet acide tel que défini; ou bien

(B) à faire entrer un composé de formule III en contact avec un agent de cétalisation pour former un premier cétal optiquement actif de formule

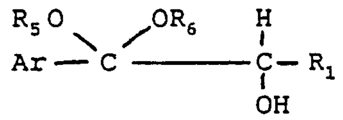

dans laquelle $R_5$ et $R_6$ sont des groupes alkyle, aryle ou aralkyle, facultativement identiques ou différents, ou forment conjointement un groupe alkylène ayant 2 à 8 atomes de carbone; á régénérer un groupe partant dans la position α du premier cétal pour former un second cétal optiquement actif de formule

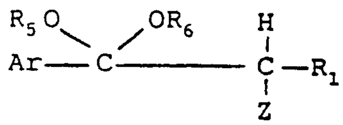

et à transposer le second cétal pour former un acide α-alcanoïque ou un ester, ortho-ester ou amide de cet acide tel que défini sous réserve que lorsque $R_5$ et $R_6$ sont des groupes alkyle, Z dans le second cétal soit autre chose qu'un groupe sulfonyloxy; ou bien

(C) à chauffer un composé optiquement actif de formule III avec un agent de cétalisation en présence d'un acide à une température élevée pendant une durée suffisante pour obtenir un acide α-arylalcanoïque tel que défini; et facultativement

(D) à hydrolyser tout ester, ortho-ester ou amide de la voie d'obtention (A) ou (B) en l'acide correspondant, et en outre à titre facultatif

(E) à convertir ledit acide α-arylalcanoïque des étapes (A) à (D) en un sel pharmaceutiquement acceptable de cet acide.

6. Procédé de production d'un cétal optiquement actif d'un composé optiquement actif de formule

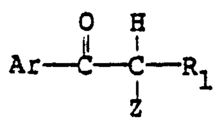

(III)

dans laquelle Ar est un groupe aryle, $R_1$ est un groupe alkyle ou cycloalkyle et Z est un groupe partant ou un groupe pouvant être transformé en un groupe partant, sous réserve que, lorsque Z est un halogène ou un groupe sulfonyloxy, le cétal représent autre chose d'un dialkylcétal dans lequel Z est un groupe hydroxy ou sulfonyloxy; procédé qui consiste à faire entrer le composé de formule III dont au moins une quantité dominante a une configuration absolue prédéterminée, en contact avec un agent de cétalisation efficace pour maintenir ladite configuration absolue prédéterminée.

7. Procédé suivant la revendication 2(A), la revendication 5(A) ou la revendication 6, dans lequel l'agent de cétalisation est un ortho-ester ou un alcool polyhydroxylique.

8. Procédé suivant la revendication 7, dans lequel l'ortho-ester un ortho-formiate de trialkyle.

9. Procédé suivant la revendication 7, dans lequel l'agent de cétalisation est l'ortho-formiate de triméthyle, l'ortho-formiate de triéthyle, l'éthylène-glycol, le triméthylène-glycol ou le 2,2-diméthyl-1,3-propanediol.

10. Procédé suivant la revendication 2(A), la revendication 2(B), la revendication 5(A) ou la revendication 5(B), dans lequel transposition est conduite par solvolyse ou en présence d'un agent doué d'affinité pour l'oxyène ou en présence d'un sel d'argent (I) d'un anion organique ou inorganique en milieu acide alcoolique.

11. Procédé suivant la revendication 10, dans lequel l'agent doué d'affinité pour l'oxygène est choisi entre des iodotrialkylsilanes, des perfluoralkylsulfonates de trialkyle et des acides de Lewis.

12. Procédé suivant la revendication 11, dans lequel l'acide de Lewis est choisi entre les acétates, propionates, benzoates, trifluorométhanesulfonates, méthanesulfonates, chlorures, iodures, bromures et sulfates du cuivre, magnésium, calcium, zinc, cadmium, baryum, mercure, étain, antimoine, bismuth, manganèse(II), fer, cobalt, nickel et palladium.

13. Procédé suivant la revendication 10, dans lequel l'agent doué d'affinité pour l'oxygène est l'iodotriméthylsilane, le trifluorométhanesulfonate de triméthylsilyle, le pentafluoromèthanesulfonate de triméthylsilyle, le chlorure d'aluminium, le bromure d'aluminium, le chlorure de zinc, le chlorure stanneux, le chlorure stannique, le chlorure de titane, le fluorure de bore, le chlorure ferrique ou le chlorure ferreux.

14. Procédé suivant la revendication 10, dans lequel le sel d'argent (I) est choisi entre le tétrafluoroborate d'argent, le carbonate d'argent, l'acétate d'argent et l'oxyde d'argent.

15. Procédé suivant la revendication 10, dans lequel la solvolyse est conduite en présence d'un solvant protique ou d'un solvant aprotique dipolaire ou en présence d'un sel d'un anion organique ou inorganique.

16. Procédé suivant la revendication 15, dans lequel le sel est choisi entre les sels de calcium, sodium, potassium et lithium des anions carbonate et bicarbonate, des anions d'acides organiques et de l'anion phosphate.

17. Procédé suivant la revendication 2(B) ou 5(B), dans lequel le composé de formule III est cétalisé au moyen d'un alcoolate, arylate ou aralkylate de métal alcalin.

18. Procédé suivant la revendication 2(C) ou la revendication 5(C), dans laquel ledit acide est l'acide sulfurique.

19. Procédé suivant la revendication 18, dans lequel l'agent de cétalisation est un orthoester.

20. Procédé suivant la revendication 19, dans lequel l'ortho-ester est un ortho-formiate de trialkyle.

21. Procédé suivant la revendication 20, dans lequel l'ortho-formiate de trialkyle est l'orthoformiate de triméthyle ou l'ortho-formiate de triéthyle.

22. Procédé suivant l'une quelconque des revendications 18 à 21, dans lequel la température élevée est d'au moins 80°C.

23. Procédé suivant l'une quelconque des revendications précédentes, dans lequel Ar est un groupe 6-méthoxy-2-naphtyle et $R_1$ est un groupe méthyle.

24. Procédé suivant l'une quelconque des revendications précédentes, dans lequel Z est choisi entre les groupes hydroxy, acétoxy, benzoyloxy, dihydropyrannyloxy, trialkylsiloxy, un halogène et un groupe de formule

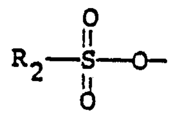

dans laquelle $R_2$ est un groupe alkyle, cycloalkyle, alcényle, alcynyle, aryle ou arylalkyle.

25. Procédé suivant la revendication 24, dans lequel Z est choisi entre des groupes alkylsulfonyloxy, notamment méthanesulfonyloxy, arylsulfonyloxy, notamment benzènesulfonyloxy et p-toluènesulfonyloxy, et aralkylsulfonyloxy.

26. Procédé suivant l'une quelconque des revendications 1 à 5 et 7 à 25 (sans surbordination à la revendication 6), dans lequel l'halogénure d'acyle, l'acylamine ou l'anhydride d'acide (dans le cas du procédé suivant la revendication 1 ou la revendication 2) ou le composé de formule III (dans le cas du procédé suivant la revendication 5) est un seul stéréo-isomère en l'absence essentielle de tout autre stéréo-isomère du composé, ou un seul stéréo-isomère de configuration absolue prédéterminée, et les réactions sont conduites dans des conditions qui évitent une racémisation notable, de sorte que le composé obtenu comme produit final désiré est un seul stéréo-isomère optiquement actif en l'absence essentielle de tout autre stéréo-isomère de ce composé, ou un seul stéréo-isomère de configuration absolue prédéterminée.

27. Procédé suivant la revendication 26, dans lequel Ar est un groupe 6-méthoxy-2-naphthyle et $R_1$ est un groupe méthyle, et au moins le produit final désiré a la configuration (S).

28. Matière comprenant un seul stéréo-isomère d'un cétal cyclique d'un composé de formule

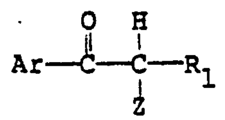

(III)

en l'absence essentielle de tout autre stéréo-isomère dudit composé, formule dans laquelle Ar est un groupe 6-méthoxy-2-naphtyle substitué ou non substituté, $R_1$ est un groupe alkyle ou cycloalkyle et Z est un groupe partant ou un groupe pouvant être transfoirmé en un groupe partant choisi entre un halogène, un groupe acétoxy, benzoyloxy, dihydropyrannyloxy ou trialkylsiloxy.

29. Matière suivant la revendication 28, dans laquelle Z est un halogène.

30. Matière suivant la revendication 29, dans laquelle Z est le brome.

31. Matière suivant l'une quelconque des revendications 28 à 30, dans laquelle Ar est le groupe 5-bromo-6-méthoxy-2-naphthyle.

32. Cétal cyclique optiquement actif ayant une configuration (S) absolue de formule:

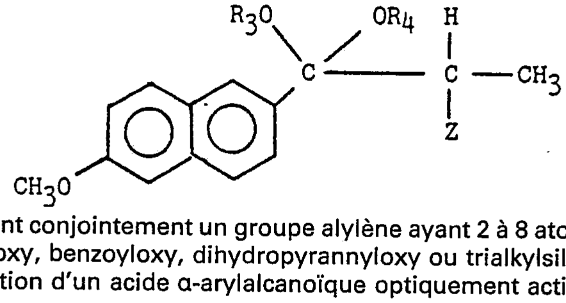

dans laquelle $R_3$ et $R_4$ forment conjointement un groupe alylène ayant 2 à 8 atomes de carbone est Z est un halogène, une groupe acétoxy, benzoyloxy, dihydropyrannyloxy ou trialkylsiloxy.

33. Procédé de préparation d'un acide α-arylalcanoïque optiquement actif de formule

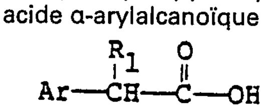

dans laquelle Ar et $R_1$ sont tels que définis dans la revendication 28, ou d'un sel, ester, ortho-ester ou amide pharmaceutiquement accceptable de cet acide; procédé qui comprend la transposition d'un cétal cyclique optiquement actif suivant l'une quelconque des revendications 28 à 32.

34. Procédé suivant la revendication 33, dans lequel Ar est le groupe 6-méthoxy-2-naphtyle et $R_1$ est le groupe méthyle.

35. Procédé suivant la revendication 34, dans lequel est préparé l'acide (S)-2-(6-méthoxy-2-naphtyl)-propionique ou un sel, ester, ortho-ester ou amide pharmaceutiquement acceptable de cet acide.

36. Procédé suivant la revendication 35, dans lequel est prépare l'acide (S)-2-(6-méthoxy-2-naphtyl)-propionique ou son sel de sodium.

37. Matière comprenant un seul stéréo-isomère d'un composé de formule

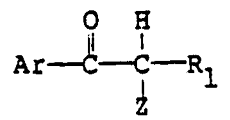

(III)

en l'absence essentielle de tout autre stéréo-isomère dudit composé, formule dans laquelle Ar est le groupe 6-méthoxy-2-naphtyle, $R_1$ est un groupe alkyle ou cycloalkyle et Z est un groupe partant ou un groupe pouvant être transformé en un groupe partant choisi entre des groupes hydroxy, acétoxy, benzoyloxy, dihydropyrannyloxy, trialkylsiloxy ou un groupe de formule

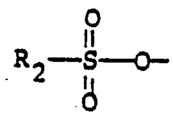

dans laquelle $R_2$ est un groupe cycloalkyle, alcényle ou alcynyle.

38. Matière comprenant un seul stéréo-isomère d'un composé de formule

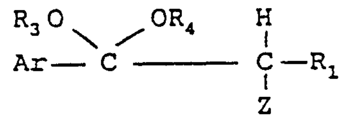

en l'absence essentielle de tout autre stéréo-isomère dudit composé, formule dans laquelle Ar est un groupe 6-méthoxy-2-naphthyle, $R_1$ est un groupe alkyle ou cycloalkyle, $R_3$ et $R_4$ sont des groupes alkyle facultativement identiques ou différents ou forment conjointement un groupe alkylène ayant 2 à 8 atomes de carbone et Z est un groupe partant ou un groupe apte à être transformé en un groupe partant, notamment un groupe choisi entre un halogène, un groupe acétoxy, benzoyloxy, dihydropyrannyloxy ou trialkylsiloxy.

39. Matière suivant la revendication 38, dans laquelle $R_3$ et $R_4$ sont chacun un groupe méthyle ou éthyle ou forment conjointement un radical éthylène, triméthylène ou 2,2-diméthyltriméthylène.

40. Matière suivant l'une quelconque des revendications 37 à 39, dans laquelle Z est choisi entre des radicaux hydroxy, chloro, bromo, triméthylsiloxy ou triéthylsiloxy.

41. Matière suivant la revendication 38, dans laquelle le seul stéréo-isomère est choisi entre
le (R) ou (S)-2-chloro-1,1-diméthoxy-1-(6-méthoxy-2-naphtyl)propane,
le (R) ou (S)-2-bromo-1,1-diméthoxy-1-(6-méthoxy-2-naphtyl)propane,
le (R) ou (S)-2-chloro-1,1-diéthoxy-1-(6-méthoxy-2-naphtyl)propane,
le (R) ou (S)-2-bromo-1,1-diéthoxy-1-(6-méthoxy-2-naphtyl)propane,
l'éthylène-acétal de la (R) ou (S)-2-chloro-1-(6-méthoxy-2-naphtyl)propane-1-one,
l'éthylène-acétal de la (R) ou (S)-2-bromo-1-(6-méthoxy-2-naphtyl)propane-1-one,

42. Matière suivant l'une quelconque des revendications 37 à 41, dans laquelle ledit stéréo-isomère a la configuration (S).

43. Matière suivant l'une quelconque des revendications 39, 40 et 42, dans laquelle Ar est un groupe 6-méthoxy-2-naphtyle et $R_1$ est un groupe méthyle.